# EUROPEAN PATENT APPLICATION

(11) **EP 4 310 151 A1**
(43) Date of publication of application: **24.01.2024**
(21) Application number: 22771272.6
(22) Date of filing: 10.03.2022
(51) Int. Cl.: C08L 83/14, A61K 8/891, A61Q 1/00, C08G 77/50, C08L 83/05, C08L 83/07

(54) **AQUEOUS DISPERSION OF SILICONE RUBBER PARTICLES, AND COSMETIC PREPARATION**

(30) Priority: 15.03.2021 JP 2021041362
(71) Applicant: SHIN-ETSU CHEMICAL CO., LTD., Tokyo 1000005 (JP)
(72) Inventor: INOKUCHI Yoshinori, Annaka-shi, Gunma 379-0224 (JP); KONISHI Masayuki, Tokyo 100-0005 (JP); HORIGUCHI Ryuji, Annaka-shi, Gunma 379-0224 (JP)
(74) Representative: Cabinet Nony
(86) International application number: PCT/JP2022/010614
(87) International publication number: WO 2022/196521

(57) **Abstract**

[Problem]

An object of the present invention is to provide an aqueous dispersion of silicone rubber spherical particles having excellent oil absorptiveness, relative to certain oil materials, specifically hydrocarbon oils or ester oils, and cosmetics to which the aqueous dispersion is added, having excellent use feeling, finish, adherence, and sebum resistance.

[Solutions to the problems]

An aqueous dispersion of silicone rubber particles comprising (A) a silicone rubber particle being an addition reaction product of (A-1) an organohydrogenpolysiloxane and (A-2) an organopolysiloxane having a monovalent olefin hydrocarbon group, wherein at least one of component (A-1) and component (A-2) has monovalent aliphatic saturated hydrocarbon groups having 6 to 30 carbon atoms, each bonded to a silicon atom, in a percentage of 5% to 70%, based on the total number of all substituents each bonded to a silicon atom, in an amount of 5 % by mass to 80 % by mass, based on a total mass of the aqueous dispersion; (B) at least one surfactant in an amount of 0.01 % by mass to 15 % by mass, based on a total mass of the aqueous dispersion; and (C) water in an amount of 19 % by mass to 94 % by mass, based on a total mass of the aqueous dispersion.

## Description

### TECHNICAL FIELD

The present invention relates to an aqueous dispersion of crosslinked silicone rubber particles having a long-chain alkyl group and, specifically, relates to cosmetics comprising the aqueous dispersion.

### BACKGROUND OF THE INVENTION

Conventionally, makeup cosmetics have been required to have persistence of makeup effects. However, there are problems in that, following the application of makeup cosmetics to a skin, the makeup effects deteriorate over time due to the sebum secreted from the skin. Examples of methods to solve these problems include applying a makeup that suppresses sebum as a base, applying makeup cosmetics resistant to sebum, and applying a touch-up makeup that suppresses sebum.

A method of selectively removing sebum by spraying over makeup cosmetics is disclosed in Patent Literature 1. However, in order to physically remove the sebum, it needs to be wiped off with a tissue, resulting in transfer of makeup components to the tissue side or smudged makeup in some cases.

Meanwhile, a technique that controls sebum using oil-absorbing powder is disclosed in Patent Literatures 2 and 3. However, the oil-absorbing powder used here has oil absorption amount relative to an oil material included in cosmetics such as silicone oil, and has poor oil absorptiveness relative to hydrocarbon oils and ester oils that are sebum-like components. Since the oil-absorbing powder having an irregular powder form is used, powder having less oil absorptiveness needs to be used together as an agent for improving feeling, resulting in the need for excessively adding the powder to the entire cosmetic. Further, while the use of silicone powder having oil absorptiveness relative to a wide range of oil materials has been disclosed in Patent Literature 4, the surfaces of particles are coated with hydrophobic polyorganosilsesquioxane, making it difficult to add it to aqueous preparations.

Further, a technique in which silicone rubber powder is preliminarily dispersed in a water phase to make a dispersion liquid is disclosed in Patent Literature 5. However, these dispersing elements are a technique to improve use feeling such as slipperiness, and a study on oil absorption of sebum has not been performed.

### PRIOR LITERATURES

### PATENT LITERATURES

Patent Literature 1: Japanese Patent Application Laid-Open No. 2001-294522
Patent Literature 2: Japanese Patent Application Laid-Open No. 2002-114665
Patent Literature 3: Japanese National Phase Publication No.2017-501993
Patent Literature 4: Japanese Patent Application Laid-Open No. 2011-105662
Patent Literature 5: Japanese Patent Application Laid-Open No. Hei 10-139624/1998

### DISCLOSURE OF THE INVENTION

### PROBLEMS TO BE SOLVED BY THE INVENTION

The present invention has been made in view of the above circumstances. An object of the present invention is to provide an aqueous dispersion of silicone rubber spherical particles having excellent oil absorptiveness, relative to certain oil materials, specifically hydrocarbon oils or ester oils, and cosmetics, to which the aqueous dispersion is added, having excellent use feeling, finish, adherence, and sebum resistance.

### SOLUTIONS TO THE PROBLEMS

The present inventors conducted keen researches to achieve the above object and has found that the above object may be achieved with an aqueous dispersion of silicone rubber particles having a certain long-chain hydrocarbon group described below, thus resulting in the present invention.

That is, the present invention provides an aqueous dispersion of silicone rubber particles described below and cosmetics comprising the aqueous dispersion.

An aqueous dispersion of silicone rubber particles, comprising
(A) the silicone rubber particle being an addition reaction product of (A-1) an organohydrogenpolysiloxane and (A-2) an organopolysiloxane having a monovalent olefin hydrocarbon group, wherein at least one of components (A-1) and (A-2) has a monovalent aliphatic saturated hydrocarbon group having 6 to 30 carbon atoms, bonded to a silicon atom, in a percentage of 5 to 70%, based on the total number of all substituents each bonded to a silicon atom,
   in an amount of 5 % by mass to 80 % by mass, based on a total mass of the aqueous dispersion;
(B) at least one surfactant in an amount of 0.01 to 15 % by mass, based on a total mass of the aqueous dispersion; and
(C) water in an amount of 19 to 94 % by mass, based on a total mass of the aqueous dispersion.

### EFFECTS OF THE INVENTION

The aqueous dispersion of silicone rubber particles of the present invention has excellent oil absorptiveness relative to certain oil materials, specifically hydrocarbon oils or ester oils. By adding the aqueous dispersion to a cosmetic, excellent use feeling, finish, adherence, sebum resistance, and dispersibility may be provided.

### DETAILED DESCRIPTION OF THE INVENTION

The following describes the present invention in detail.

### [Aqueous dispersion of silicone rubber particles]

### (A) Silicone rubber particles

Component (A) represents silicone rubber spherical particles. The silicone rubber particle is an addition reaction product of (A-1) an organohydrogenpolysiloxane and (A-2) a monovalent olefin hydrocarbon group-containing organopolysiloxane. In the silicone rubber particle, at least one of component (A-1) and component (A-2) has monovalent aliphatic saturated hydrocarbon groups having 6 to 30 carbon atoms, each bonded to a silicon atom, in a percentage of 5% to 70% based on the total number of all substituents each bonded to a silicon atom.

The organohydrogenpolysiloxane (A-1) is preferably an organohydrogenpolysiloxane having two or more hydrogen atoms each bonded to a silicon atom within one molecule and represented by an average composition formula (1): R¹ₐH_{b}SiO_{(4-a-b)/2} (1)
wherein R¹ is, independently of each other, an unsubstituted or substituted monovalent hydrocarbon group having 1 to 30 carbon atoms free from an aliphatic unsaturated bond, and *a* and *b* are the positive number satisfying equations 0 < *a* < 3, 0 < *b* < 3, and 0.1 ≤ *a* + *b* ≤ 3*.*

The monovalent olefin hydrocarbon group-containing organopolysiloxane (A-2) is preferably an organopolysiloxane having two or more monovalent olefin hydrocarbon groups in one molecule and represented by an average composition formula (2): R²_{c}R³_{d}SiO_{(4-c-d)/2} (2)
wherein R² is, independently of each other, an unsubstituted or substituted monovalent hydrocarbon group having 1 to 30 carbon atoms free from an aliphatic unsaturated bond, R³ is, independently of each other, a monovalent olefin hydrocarbon group having 2 to 6 carbon atoms, and *c* and *d* are a positive number satisfying equations 0 < c <3, 0 < *d* ≤ 3, and 0.1 ≤ *c* + *d* ≤ 3. The monovalent olefin hydrocarbon group is preferably an alkenyl group having 2 to 6 carbon atoms such as a vinyl group, allyl group, propenyl group, butenyl group, pentenyl group, or hexenyl group, and more preferably a vinyl group.

The amount of component (A-2) relative to that of component (A-1) is an amount such that a ratio of the number of monovalent olefin hydrocarbon groups in component (A-2) per number of hydrogen atom bonded to a silicon atom in component (A-1) is 0.5 to 2, and that at least one of component (A-1) and component (A-2) has monovalent hydrocarbon groups having 6 to 30 carbon atoms in a percentage of 5% to 70% of the total number of all substituents each bonded to a silicon atom.

Noted that, the combination of the organohydrogenpolysiloxane (A-1) having only two SiH groups and the organopolysiloxane (A-2) having only two olefin hydrocarbon groups is excluded. That is, when component (A-1) has two SiH groups, at least one of component (A-2) is an organosiloxane having three or more monovalent olefin hydrocarbon groups. Further, when component (A-2) has two monovalent olefin hydrocarbon groups, at least one of component (A-1) is an organohydrogensiloxane having three or more SiH groups.

The silicone rubber particles may have a volume average particle diameter of from 0.1 µm to 100 µm, and preferably from 1 µm to 40 µm. If the volume average particle diameter is smaller than the afore-mentioned upper limit, the resulting silicone microparticles are less likely to exhibit silkiness and smoothness. If the volume average particle diameter is larger than the afore-mentioned upper limit, the degree of silkiness and smoothness of the resulting silicone microparticles tends to deteriorate, and a feeling of grittiness may occur. The volume average particle diameter is determined using a Coulter counter method (electrical resistance method).

The silicone rubber particles are preferably spherical. In the present invention, the term "spherical" includes not only microparticles having a perfectly spherical shape, but also microparticles having deformed spherical shapes in which the average of the ratio of the longest axis length to the shortest axis length (aspect ratio) is typically within a range from 1 to 4, preferably from 1 to 2, more preferably from 1 to 1.6, and still more preferably from 1 to 1.4. The shapes of the microparticles may be confirmed by inspecting the microparticles under an optical microscope, electron microscope, and the like. Being spherical is preferable in terms of use feeling.

The silicone rubber that constitutes the silicone rubber particles preferably exhibits no stickiness, and preferably has a rubber hardness, determined using an Asker Durometer Type C prescribed in the Society of Rubber Science and Technology, Japan Standards (SRIS) 0101, that is within a range from 10 to 95, and more preferably within a range from 20 to 85. Provided the rubber hardness is within a range from 10 to 95, superior feelings of smoothness and softness may be obtained.

The silicone rubber particles (A) of the present invention may absorb 30 parts by mass or more, preferably 40 parts by mass or more, and further preferably 70 parts by mass or more, of hydrocarbon oils or ester oils relative to 100 parts by mass of the silicone rubber. If the absorbing amount of the hydrocarbon oils and the ester oils that are sebum-like components is less than 30 parts by mass, the effect in suppressing changes in the makeup cosmetics properties and the effect in suppressing greasiness, stickiness, and oily film feeling of cosmetics comprising at least one of liquid oils composed of hydrocarbon oils and ester oils tend to weaken. A greater absorbing amount of oily substance is more preferred and, therefore, the upper limit for the absorbing amount is not particularly limited. For practical reasons, the absorbing amount may be, for example, 500 parts by mass or less, and particularly 300 parts by mass or less.

The hydrocarbon oils and ester oils described above, which represent oily substances, are used as raw materials in cosmetic products. Examples of the hydrocarbon oils include linear or branched hydrocarbon oils, and the hydrocarbon oils may be both volatile hydrocarbon oils and non-volatile hydrocarbon oils. Examples of the hydrocarbon oils include squalanes such as synthetic squalane and plant-based squalane, squalene, light liquid isoparaffin, liquid paraffin, liquid isoparaffin, and hydrogenated isopolybutene. Examples of the ester oils include diethylhexyl succinate, diethylhexyl sebacate, dibutyloctyl sebacate, diisocetyl adipate, diisostearyl malate, di-2-heptylundecyl adipate, propylene glycol dicaprate, neopentyl glycol dicaprate, trimethylolpropane triethylhexanoate, pentaerythritol tetraethylhexanoate, ethyl linoleate, isopropyl isostearate, isopropyl linoleate, butyl stearate, octyl palmitate, ethylhexyl palmitate, ethylhexyl stearate, decyl oleate, myristyl myristate, cetyl ethylhexanoate, cetyl octanoate, cetyl palmitate, isocetyl dimethyloctanoate, isocetyl myristate, isocetyl palmitate, isocetyl stearate, isocetyl isostearate, isostearyl palmitate, octyldodecyl neopentanoate, octyldodecyl oleate, and octyldodecyl myristate. Particularly preferably, the silicone rubber particles (A) of the present invention may absorb 30 parts by mass or more, preferably 40 parts by mass or more, and further preferably 70 parts by mass or more, of squalane or ethylhexyl palmitate relative to 100 parts by mass of the silicone rubber particles.

Preferably, the silicone rubber particle (A) of the present invention is a cured product of a silicone composition comprising the organohydrogenpolysiloxane (A-1), the monovalent olefin hydrocarbon group-containing organopolysiloxane (A-2), and a platinum group metal-based catalyst (A-3). The following describes each component in more detail.

Component (A-1) is preferably an organohydrogenpolysiloxane having hydrogen atoms (hereinafter referred to as "SiH groups" in some cases) each bonded to a silicon atom in one molecule, represented by the average composition formula (1) below.

R¹ₐH_{b}SiO_{(4-a-b)/2} (1)

wherein R¹ is, independently of each other, an unsubstituted or substituted monovalent hydrocarbon group having 1 to 30 carbon atoms free from an aliphatic unsaturated bond, and *a* and *b* are a positive number satisfying equations 0 < *a* < 3, 0 < *b* < 3, and 0.1 ≤ *a* + *b* ≤ 3. One of the organopolysiloxanes represented by the average composition formula (1) may be used alone, or two or more of them may be used in combination.

The *a* and *b* are preferably a positive number satisfying equations 0 < *α* ≤ 2.295, 0.005 ≤ *b* ≤ 2.3, and 0.5 ≤ *a* + *b* ≤ 2.3.

The R¹ is an unsubstituted or substituted monovalent hydrocarbon group having 1 to 30 carbon atoms, preferably 1 to 22 carbon atoms, and more preferably 1 to 18 carbon atoms, free from an aliphatic unsaturated bond. Examples of R¹ include alkyl groups such as a methyl group, an ethyl group, a propyl group, a butyl group, a pentyl group, a hexyl group, a heptyl group, an octyl group, a decyl group, an undecyl group, a dodecyl group, a tetradecyl group, a pentadecyl group, a hexadecyl group, a heptadecyl group, an octadecyl group, a nonadecyl group, an icosyl group, a henicosyl group, a docosyl group, a tricosyl group, a tetracosyl group, or a triacontyl group; aryl groups such as a phenyl group, a tolyl group or a naphthyl group; aralkyl groups such as a benzyl group or a phenethyl group; cycloalkyl groups such as a cyclopentyl group, a cyclohexyl group, or a cycloheptyl group; and monovalent hydrocarbon groups in which some or all of the hydrogen atoms bonded to carbon atoms in any of the above groups have been substituted with one or both of an atom such as a halogen atom (such as a fluorine atom, a chlorine atom, a bromine atom, or a iodine atom), and a substituent such as an acryloyloxy group, a methacryloyloxy group, an epoxy group, a glycidoxy group, or a carboxyl group.

The viscosity of the organohydrogenpolysiloxane (A-1) is preferably 100,000 mm²/s or less, and is more preferably 10,000 mm²/s or less. Provided the viscosity is 100,000 mm²/s or less, the preparation method described below may be used to particularly easily provide silicone microparticles having a narrow particle size distribution. Although there are no particular limitations on the lower limit for the viscosity, for practical reasons, the viscosity may be, for example, 0.4 mm²/s or more, and particularly 2 mm²/s or more. Furthermore, the structure of the organohydrogenpolysiloxane (A-1) may be a linear, cyclic, or branched structure, and a linear structure is particularly preferred.

Particularly preferably, the organohydrogenpolysiloxane (A-1) is represented by the following formula (1').

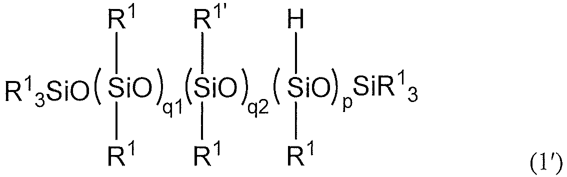

In the formula (1'), R^{1'} is a monovalent aliphatic saturated hydrocarbon group having 6 to 30 carbon atoms. R¹ is a substituent other than the R^{1'} among the above-described R¹, preferably an alkyl group having 1 to 6 carbon atoms, and more preferably a methyl group. *q1* and *q2* are integers of 1 or more, *p* is an integer of 2 or more, and *q1, q2* and *p* are integers with which the viscosity of the organohydrogenpolysiloxane is 100,000 mm²/s or less, preferably 10,000 mm²/s or less, and 0.4 mm²/s or more, preferably 2 mm²/s or more. Furthermore, *q*2 is an integer such that the percentage of the number of R^{1'} is 5% to 70%, preferably 8% to 60%, based on the total number of all substituents (R¹ and R^{1'}) each bonded to a silicon atom. *q1* is preferably an integer of 1 to 60, more preferably an integer of 10 to 50, q2 is preferably an integer of 1 to 50, more preferably an integer of 5 to 30, and *p* is preferably an integer of 2 to 20, more preferably an integer of 3 to 10. In the present invention, the bonding order of the siloxane units is not limited to the above, and may be random or may form block units.

Component (A-2) is preferably an organopolysiloxane having two or more monovalent olefin hydrocarbon groups in one molecule, represented by the average composition formula (2): R²_{c}R³_{d}SiO_{(4-c-d)/2} (2)
wherein R² is, independently of each other, an unsubstituted or substituted monovalent hydrocarbon group having 1 to 30 carbon atoms free from an aliphatic unsaturated bond, R³ is, independently of each other, a monovalent olefin hydrocarbon group having 2 to 6 carbon atoms, and *c* and *d* are a positive number satisfying equations 0 < *c* <3, 0 < *d* ≤ 3, and 0.1 ≤ *c* + *d* ≤ 3*.* One of the organopolysiloxanes represented by the above average composition formula (2) may be used alone, or two or more of them may be used in combination.

The ***c*** and ***d*** are preferably a positive number satisfying equations 0 < *c* ≤ 2.295, 0.005 ≤ *d* ≤ 2.3, 0.5 ≤ *c* + *d* ≤ 2.3.

The R² is an unsubstituted or substituted monovalent hydrocarbon group having 1 to 30 carbon atoms, preferably 1 to 22 carbon atoms, and more preferably 1 to 18 carbon atoms, free from an aliphatic unsaturated bond. Examples of the R² include the above-described groups exemplified for R¹. In at least one of component (A-1) and component (A-2), 5% to 70%, preferably 8 mol% to 60 mol%, of the total number of all substituents (that is, R¹ in the formula (1) or R² and R³ in the formula (2)) each bonded to a silicon atom represent monovalent aliphatic saturated hydrocarbon groups having 6 to 30 carbon atoms, preferably 8 to 20 carbon atoms, and more preferably 10 to 15 carbon atoms. If the proportion of the long-chain monovalent aliphatic saturated hydrocarbon group is less than the afore-mentioned lower limit, the absorbing amount of the above oily substances tends to decrease. If the proportion exceeds the afore-mentioned upper limit, the reactivity of the organohydrogenpolysiloxane of component (A-1) and the organopolysiloxane having olefin hydrocarbon groups of component (A-2) tends to deteriorate, and the rubber hardness of the resulting cured product tends to decrease.

The R³ is, independently of each other, a monovalent olefin hydrocarbon group having 2 to 6 carbon atoms. Examples of the R³ include alkenyl groups having 2 to 6 carbon atoms such as a vinyl group, an allyl group, a propenyl group, a butenyl group, a pentenyl group, or a hexenyl group. From an industrial perspective, a vinyl group is preferred.

The viscosity of component (A-2) is preferably 100,000 mm²/s or less, and is more preferably 10,000 mm²/s or less. When the viscosity is 100,000 mm²/s or less, the preparation method described below may be used to particularly easily provide silicone microparticles having a narrow particle size distribution. Although there are no particular limitations on the lower limit for the viscosity, for practical reasons, the viscosity may be, for example, 0.7 mm²/s or more, and particularly 2 mm²/s or more. Furthermore, although the structure of the organopolysiloxane (A-2) may be a linear, cyclic, or branched structure, a linear structure is particularly preferred. In addition, although the olefin hydrocarbon groups may be bonded to any of silicon atoms at side chains and terminals without particular restriction of the bonding positions of the olefin hydrocarbon groups, it is particularly preferred that the olefin hydrocarbon groups are bonded to the silicon atoms at both terminals of a linear organopolysiloxane.

Particularly preferably, the organopolysiloxane (A-2) is represented by the following formula (2').

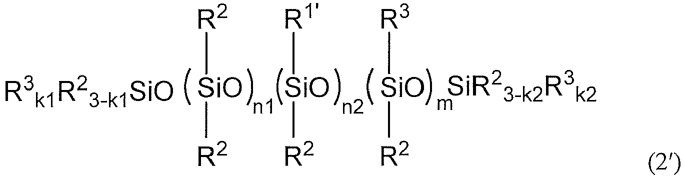

In the formula (1'), R^{1'} is a monovalent aliphatic saturated hydrocarbon group having 6 to 30 carbon atoms. R² is a substituent other than the R^{1'} among the above-described R², preferably an alkyl group having 1 to 6 carbon atoms, and more preferably a methyl group. R³ is, as described, preferably an alkenyl group having 2 to 6 carbon atoms, and more preferably a vinyl group. *n1* is an integer of 1 or more, *n2* is an integer of 0 or more, *m* is an integer of 0 or more, and *k1* and *k2* are each an integer of 0 to 3 and, provided that, *m*+*k1*+*k*2 is an integer of 2 or more. Preferably, *k1* and *k2* are both 1. Furthermore, *n1, q2,* and *m* are integers with which the viscosity of the organopolysiloxane is 100,000 mm²/s or less, preferably 10,000 mm²/s or less and 0.4 mm²/s or more, preferably 2 mm²/s or more. Additionally, *n2* is preferably an integer such that percentage of the number of R¹ is 5% to 70%, preferably 8% to 60%, based on the total number of all substituents (R² and R^{1'}) each bonded to a silicon atom. *n1* is preferably an integer of 1 to 40, more preferably an integer of 5 to 30. *n2* is preferably an integer of 0 to 50, more preferably an integer of 0 or 1 to 20. *m* is preferably an integer of 0 to 10, more preferably an integer of 0 or 1 to 5. In the present invention, the bonding order of the siloxane units is not limited to the above, and may be random or may form block units.

From the viewpoint of reactivity, in component (A-1) and component (A-2), substituents other than monovalent aliphatic saturated hydrocarbon groups having 6 to 30 carbon atoms (R^{1'}) are preferably methyl groups. Both component (A-1) and component (A-2) may have monovalent aliphatic saturated hydrocarbon groups having 6 to 30 carbon atoms (R^{1'}) in the respective proportions described above. Preferably, any one of component (A-1) and component (A-2) has monovalent hydrocarbon groups having 6 to 30 carbon atoms each bonded to a silicon atom in a percentage of 5% to 70%, preferably 8% to 60%, based on the total number of all substituents each bonded to a silicon atom. Further preferably, only component (A-1) has monovalent aliphatic saturated hydrocarbon groups having 6 to 30 carbon atoms each bonded to a silicon atom in a percentage of 5% to 70%, preferably 8% to 60%, based on the total number of all substituents each bonded to a silicon atom.

As described above, component (A-1) is an organohydrogenpolysiloxane having two or more hydrogen atoms each bonded to a silicon atom in one molecule, and component (A-2) is an organopolysiloxane having two or more monovalent olefin hydrocarbon groups in one molecule. The combination of the organohydrogenpolysiloxane (A-1) having only two SiH groups and the organopolysiloxane (A-2) having only two olefin hydrocarbon groups is excluded. This is because a cured product from the combination of the organohydrogensiloxane having only two SiH groups as component (A-1) and the organopolysiloxane having only two olefin hydrocarbon groups as component (A-2) has stickiness, and silicone rubber cannot be obtained. That is, when component (A-1) has only two SiH groups, at least one of component (A-2) is an organosiloxane having three or more olefin hydrocarbon groups. Further, when component (A-2) has only two olefin hydrocarbon groups, at least one of component (A-1) is an organohydrogensiloxane having three or more SiH groups.

The amount of component (A-2) relative to component (A-1) is an amount such that a ratio of the number of monovalent olefin hydrocarbon groups in component (A-2) per number of SiH group in component (A-1) is 0.5 to 2, preferably 0.7 to 1.5. If the amount of component (A-2) is such that gives the ratio of the number of monovalent olefin hydrocarbon groups less than the afore-mentioned lower limit or more than the afore-mentioned upper limit, the resulting silicone rubber cured product tends to have stickiness, and also tends to exhibit overly higher reaction activity.

The platinum group metal-based catalyst (A-3) is a catalyst for promoting the addition reaction between the SiH groups within component (A-1) and the monovalent olefin hydrocarbon groups within component (A-2). One kind of component (A-3) may be used alone, or two or more kinds may be used in combination.

Examples of component (A-3) may be well-known catalysts used in hydrosilylation reactions. Examples component (A-3) include platinum group metals such as platinum (including platinum black), rhodium, and palladium; platinum chlorides, chloroplatinic acids, and chloroplatinates such as H₂PtCl₄·kH₂O, H₂PtCl₆·kH₂O, NaHPtCl₆·kH₂O, KHPtCl₆·kH₂O, Na₂PtCl₆·kH₂O, K₂PtCl₄·kH₂O, PtCl₄·kH₂O, PtCl₂, and Na₂HPtCl₄·kH₂O, wherein k is an integer of 0 to 6, and is preferably 0 or 6; alcohol-modified chloroplatinic acid (see U.S. Patent No. 3,220,972); platinum chlorides, complexes of a chloroplatinic acid and olefins (see U.S. Patent No. 3,159,601, U.S. Patent No. 3,159,662, and U.S. Patent No. 3,775,452); complexes of a chloroplatinic acid and a vinyl group-containing siloxane, complexes of platinum and a vinyl group-containing siloxane; a platinum group metal such as platinum black or palladium supported on a carrier such as alumina, silica, or carbon; rhodium-olefin complexes; and chlorotris(triphenylphosphine)rhodium (Wilkinson's catalyst).

The amount of component (A-3) may be an effective amount as a hydrosilylation reaction catalyst, and the amount of the platinum group metal within component (A-3) is generally from 0.1 ppm to 500 ppm, preferably from 0.5 ppm to 200 ppm, and further preferably from 1 ppm to 100 ppm, in mass conversion, relative to the total amount of the composition.

The silicone rubber particle of component (A) may further contain a silicone oil, an organosilane, an inorganic powder, an organic powder, an antioxidant, and the like.

The amount of component (A) is preferably 5 % by mass to 80 % by mass, preferably 20 % by mass to 70 % by mass, and more preferably 40 % by mass to 60 % by mass, based on the total mass of the aqueous dispersion. If the amount is less than the afore-mentioned lower limit, productivity per component (A) decreases, and the amount of the aqueous dispersion to be added to cosmetics increases, which is inefficient and unpreferable. If the amount is larger than the afore-mentioned upper limit, the viscosity of the aqueous dispersion increases, making it difficult to handle.

### (B) Surfactant

A surfactant (B) functions as a dispersant for the silicone rubber particles in the aqueous dispersion and cosmetics. As will be described below, the surfactant (B) functions also as an emulsifier for a liquid silicone at the preparation of the aqueous dispersion of the silicone rubber particles.

Component (B) is not particularly limited and may be any of a nonionic surfactant, an anionic surfactant, a cationic surfactant, and an amphoteric surfactant. One kind may be used alone, or two or more kinds may be used in combination as necessary.

Examples of the nonionic surfactant include polyoxyethylene alkyl ether, polyoxyethylene polyoxypropylene alkyl ether, polyoxyethylene alkyl phenyl ether, polyethylene glycol fatty acid ester, sorbitan fatty acid ester, polyoxyethylene sorbitan fatty acid ester, polyoxyethylene sorbit fatty acid ester, glycerin fatty acid ester, polyoxyethylene glycerin fatty acid ester, polyglycerol fatty acid ester, propylene glycol fatty acid ester, polyoxyethylene castor oil, polyoxyethylene cured castor oil, polyoxyethylene cured castor oil fatty acid ester, polyoxyethylene alkyl amine, polyoxyethylene fatty acid amide, polyoxyethylene-modified organopolysiloxane, and polyoxyethylene polyoxypropylene-modified organopolysiloxane.

Examples of the anionic surfactant include alkyl sulfate ester salt such as sodium lauryl sulfate, polyoxyethylene alkylether sulfate ester salt, polyoxyethylene alkylphenyl ether sulfate ester salt, sulfate ester salt of fatty acid alkylol amide, alkyl benzene sulfonate, polyoxyethylene alkylphenyl ether sulfonate, α-olefin sulfonate, α-sulfo fatty acid ester salt, alkylnaphthalene sulfonate, alkyldiphenylether disulfonate, alkane sulfonate, N-acyl taurine acid salt, dialkylsulfosuccinic acid salt, monoalkylsulfosuccinic acid salt, polyoxyethylenealkyl ether sulfosuccinic acid salt, fatty acid salt, polyoxyethylene alkyl ether carboxylic acid salt, N-acylamino acid salt, monoalkyl phosphoric acid ester salt, dialkyl phosphoric acid ester salt, and polyoxyethylene alkylether phosphoric acid ester salt.

Examples of the cationic surfactant include alkyl trimethyl ammonium salt, dialkyl dimethyl ammonium salt, polyoxyethylene alkyl dimethyl ammonium salt, dipolyoxyethylene alkyl methyl ammonium salt, tripolyoxyethylene alkyl ammonium salt, alkyl benzyl dimethyl ammonium salt, alkyl pyridinium salt, monoalkyl amine salt, and monoalkylamide amine salt.

Examples of the amphoteric surfactant include alkyl dimethyl amine oxide, alkyl dimethyl carboxybetaine, alkylamide propyl dimethyl carboxybetaine, alkyl hydroxysulfobetaine, and alkyl carboxymethyl hydroxyethyl imidazolinium betaine.

The amount of component (B) is preferably 0.01 % by mass to 15 % by mass, preferably 0.1 % by mass to 10 % by mass, and more preferably 0.5 % by mass to 5 % by mass, based on the total mass of the aqueous dispersion. If the amount is less than 0.01 % by mass, the liquid silicone cannot be emulsified at the preparation stage, and it is difficult to obtain particles having a narrow particle size distribution. Further, the stability of the aqueous dispersion possibly decreases. If the amount is increased to more than 15 % by mass, it does not improve the water dispersity of the silicone rubber particles, and irritation to a skin possibly may increase, which is unpreferable.

### (C) Water

Water is a dispersion medium for the silicone rubber particles (A). The amount of component (C) is 19 % by mass to 94 % by mass, preferably 30 % by mass to 80 % by mass, based on the total mass of the aqueous dispersion.

### - Method for preparing aqueous dispersion of silicone rubber particles

The aqueous dispersion of silicone rubber particles may be prepared by any known method. One possible method involves, for example, adding a surfactant (B) and water (C) to a mixed solution of an organohydrogenpolysiloxane (A-1) and an organopolysiloxane having olefin hydrocarbon groups (A-2), performing an emulsification to provide an emulsion, and then adding a platinum group metal-based catalyst (A-3) to initiate an addition reaction.

The emulsification may be conducted using a typical emulsification disperser, examples of which include high-speed rotational centrifugal radial stirrers such as a homodisper, high-speed rotational shearing stirrers such as a homomixer, high-pressure injection-type emulsification dispersers such as a homogenizer, colloid mills, and ultrasonic emulsifiers.

In those cases where the platinum group metal-based catalyst (A-3) exhibits poor dispersibility within water, the platinum group metal-based catalyst (A-3) is preferably added to the emulsion in a state dissolved in the surfactant (B). The addition reaction may be conducted at room temperature (1°C to 30°C). In those cases where the reaction does not proceed to completion, the reaction may be conducted under heating at a temperature of less than 100°C.

### [Cosmetics]

The present invention further provides cosmetics comprising the aqueous dispersion of silicone rubber particles.

The aqueous dispersion of the present invention may be added to various kinds of cosmetics. For example, the aqueous dispersion is used appropriately for cosmetics particularly applied for a skin or hair such as skincare products, makeup products, hair products, antiperspirant products, and UV-ray protective products. The amount of the aqueous dispersion is not particularly limited and may be selected for each preparation. In particular, the amount is preferably in a range such that the mass of the aqueous dispersion of silicone rubber particles is 0.1 % by mass to 50 % by mass, based on the total mass of a cosmetic.

The cosmetic may contain various kinds of components used in ordinary cosmetics to the extent that the contained components do not impair the effects of the present invention. The silicone rubber particles (A) may be picked up by drying up the aqueous dispersion of the present invention through spray drying or the like. The silicone rubber particles (A), the surfactant (B), and water (C) may be each added to the cosmetic separately. It is preferred that the aqueous dispersion comprising the silicone rubber particles (A), the surfactant (B), and water (C) is added to the cosmetic in terms of use feeling and dispersibility, and also it is preferred because processes of drying up and redispersion of component (A) are not necessary.

### [Other components]

The cosmetics of the present invention may contain various kinds of components used in ordinary cosmetics as other components. As the other components, for example, (1) an aqueous component, (2) an oil material, (3) powders other than component (A), (4) a surfactant other than component (B), (5) a crosslinked organopolysiloxane, (6) a film-forming agent, and (7) other additives may be included. One of these may be used alone, or two or more of them may be used in combination as necessary. These components are selectively used as necessary according to the type of the cosmetics, and also, the amount may be a known amount according to the type of the cosmetics.

### (1) Aqueous component

The aqueous component is not particularly limited as long as it may be added to ordinary cosmetics. Examples of aqueous component include water, lower alcohols such as ethanol, sugar alcohols such as erythritol, maltitol, xylitol, and sorbitol, polyhydric alcohols such as butylene glycol (BG), glycerin, propylene glycol (PG), dipropylene glycol (DPG), and pentylene glycol, humectants such as trimethylglycine and pyrrolidone carboxylic acid salt, and essences extracted from plants. One kind may be used alone, or two or more kinds may be used in combination as necessary. When the aqueous component is added, the amount is preferably 0.1 % by mass to 90 % by mass in a cosmetic.

### (2) Oil materials

An oil material may be added to the cosmetics of the present invention. The oil material may be volatile or non-volatile and may be in any state of solid, semisolid, or liquid at room temperature. Examples of the oil material include silicone oils, natural vegetable and animal fats and oils, semi-synthetic fats and oils, hydrocarbon oils, higher alcohols, fatty acids, ester oils, fluorine-based oil materials, and ultraviolet absorbers. When the oil material is contained, the amount of the oil material is not particularly limited but is preferably 1 % by mass to 95 % by mass, more preferably 15 % by mass to 40 % by mass of an entire cosmetic.

### - Silicone oil

Examples of the silicone oils include dimethylpolysiloxane (such as KF-96L-1cs, KF-96L-1.5cs, and KF-96L-2cs produced by Shin-Etsu Chemical Co., Ltd.), octamethyltetrasiloxane (D4), decamethylpentasiloxane (D5) (KF-995 produced by Shin-Etsu Chemical Co., Ltd.), dodecamethylhexasiloxane (D6), tristrimethylsiloxymethylsilane (TMF-1.5 produced by Shin-Etsu Chemical Co., Ltd.), caprylyl methicone, phenyl trimethicone, methylphenylpolysiloxane (KF-54 and KF-54HV produced by Shin-Etsu Chemical Co., Ltd.), diphenylsiloxy phenyl trimethicone (KF-56A produced by Shin-Etsu Chemical Co., Ltd.), methylhexylpolysiloxane, methylhydrogenpolysiloxane, linear or branched organopolysiloxane with a viscosity within a range from low to high such as dimethylsiloxane-methylphenylsiloxane copolymer, amino-modified organopolysiloxane, pyrrolidone-modified organopolysiloxane, pyrrolidone-carboxylic-acid-modified organopolysiloxane, silicone rubber such as gum-like dimethylpolysiloxane with a high degree of polymerization, gum-like amino-modified organopolysiloxane, and a gum-like dimethylsiloxane-methylphenylsiloxane copolymer, a solution of a silicone gum or rubber in a cyclic organopolysiloxane, amino acid-modified silicones, fluorine-modified silicones, a solution of silicone polymer and silicone resin,.

### - Oily component in a solid form

In the present invention, when solidification of the cosmetics is desired, waxes, hydrocarbons, esters, higher alcohols, and higher fatty acids, of a solid form at 25°C, are preferably added. Oily components in a solid form at 25°C have a melting point of preferably 40°C or higher, more preferably 60°C to 110°C. Examples of the oily components include waxes, hydrocarbons, esters, higher alcohols, and higher fatty acids. The oily components are not particularly limited as long as they are raw materials that may be added to ordinary cosmetics. Specific examples of the oily components include vegetable waxes such as carnauba wax, candelilla wax, rice wax, and Japan wax, animal waxes such as yellow beeswax and spermaceti wax, hydrocarbon waxes such as solid paraffin, polyethylene, ceresine, ozokerite, and microcrystalline wax, higher alcohols such as stearyl alcohol, behenyl alcohol and cetanol, fatty acids such as stearic acid and behenic acid, and silicone waxes such as acrylic silicone resins of acryl-silicone graft or block copolymers (such as acryl-silicone graft copolymer: KP-561P and KP-562P produced by Shin-Etsu Chemical Co., Ltd.), or derivatives thereof. One kind or two or more kinds selected from these are preferred.

### - Natural vegetable and animal fat and oil, and semi-synthetic fat and oil

Examples of the natural vegetable and animal fats and oils, and semi-synthetic fats and oils include avocado oil, linseed oil, almond oil, ibota wax, perilla oil, olive oil, cacao butter, kapok wax, kaya oil, carnauba wax, liver oil, candelilla wax, refined candelilla wax, beef tallow, bovine hoof oil, bovine bone fat, hardened beef tallow, apricot kernel oil, spermaceti wax, hardened oils, wheat germ oil, sesame oil, rice germ oil, rice bran oil, sugarcane wax, sasanqua oil, safflower oil, shea butter, paulownia oil, cinnamon oil, jojoba wax, squalane, squalene, shellac wax, turtle oil, soybean oil, tea seed oil, camellia oil, evening primrose oil, corn oil, lard, rapeseed oil, Japanese tung oil, rice bran wax, germ oil, horse fat, persic oil, palm oil, palm kernel oil, castor oil, hardened castor oil, methyl esters of castor oil fatty acids, sunflower oil, grape seed oil, bayberry wax, jojoba oil, macadamia nut oil, yellow beeswax, mink oil, mealowfoam oil, cottonseed oil, cotton wax, Japan wax, sumac kernel oil, montan wax, coconut oil, hardened coconut oil, coconut fatty acid triglyceride, mutton tallow, peanut oil, lanolin, liquid lanolin, reduced lanolin, lanolin alcohol, hardened lanolin, lanolin acetate, acetylated lanolin alcohol, isopropyl esters of lanolin fatty acids, polyoxyethylene lanolin alcohol ether, polyoxyethylene lanolin alcohol acetate, lanolin fatty acid polyethylene glycol, polyoxyethylene hydrogenated lanolin alcohol ether, and egg yolk oil.

### - Oily component in a liquid form

Examples of oily components in a liquid form include hydrocarbon oils, higher fatty acids, higher alcohols, esters, silicone oils, and fluorine-based oil materials.

### - Hydrocarbon oil

Examples of the hydrocarbon oils include linear or branched hydrocarbon oils, and the hydrocarbon oils may be volatile hydrocarbon oils or non-volatile hydrocarbon oils. Specific examples of the hydrocarbon oils include α-olefin oligomer, light isoparaffin, isododecane, isohexadecane, light liquid isoparaffin, squalane, synthetic squalane, plant-based squalane, squalene, liquid paraffin, liquid isoparaffin, polyisobutylene, hydrogenated polyisobutene, and vaseline.

### - Higher fatty acid

Examples of the higher fatty acids include oleic acid, linoleic acid, linolenic acid, arachidonic acid, eicosapentaenoic acid (EPA), docosahexaenoic acid (DHA), isostearic acid, and 12-hydroxystearic acid.

### - Higher alcohol

Examples of the higher alcohols include alcohols having preferably 6 or more carbon atoms. Specific examples of the higher alcohols include oleyl alcohol, isostearyl alcohol, 2-decyltetradecinol, cholesterol, phytosterol, polyoxyethylenecholesterol ether, monostearylglyceryl ether (batyl alcohol), and monooleylglyceryl ether (selachyl alcohol).

### - Ester

Examples of the esters include diisobutyl adipate, 2-hexyldecyl adipate, di-2-heptylundecyl adipate, N-alkyl glycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dioctanoate, neopentyl glycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isononyl isononanoate, isotridecyl isononanoate, isopropyl palmitate, 2-ethylhexyl palmitate, 2-hexyldecyl palmitate, 2-heptylundecyl palmitate, cholesteryl 12-hydroxystearate, fatty acid esters of dipentaerythritol, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate, isopropyl lauroyl sarcosinate, and diisostearyl malate; and glyceride oils such as acetoglyceryl, glyceryl triisooctanoate, glyceryl triisostearate, glyceryl triisopalmitate, glyceryl tribehenate, glyceryl monostearate, glyceryl di-2-heptylundecanoate, glyceryl trimyristate, and diglyceryl myristate isostearate.

### - Fluorine-based oil material

Examples of the fluorine-based oil materials include perfluoropolyethers, perfluorodecalin, and perfluorooctane.

### - Ultraviolet absorber

Examples of the ultraviolet absorbers include homomenthyl salicylate, octocrylene, 4-tert-butyl-4'-methoxydibenzoyl methane, 4-(2-β-glucopyranosiloxy)propoxy-2-hydroxybenzophenone, octyl salicylate, 2-[4-(diethylamino)-2-hydroxybenzoyl] hexyl benzoate, dihydroxydimethoxybenzophenone, sodium dihydroxydimethoxybenzophenone disulfonate, dihydroxybenzophenone, dimethicodiethylbenzal malonate, 1-(3,4-dimethoxyphenyl)-4,4-dimethyl-1,3-pentanedione, 2-ethylhexyl dimethoxybenzylidene dioxoimidazolidine propionate, tetrahydroxy benzophenone, terephthalylidene dicamphor sulfonic acid, 2,4,6-tris[4-(2-ethylhexyloxycarbonyl)anilino]-1,3,5-triazine, methylbis(trimethylsiloxy)silylisopentyl trimethoxycinnamate, drometrizole trisiloxane, 2-ethylhexyl p-dimethylaminobenzoate, isopropyl p-methoxycinnamate, 2-ethylhexyl p-methoxycinnamate, 2,4-bis-[{4-(2-ethylhexyloxy)-2-hydroxy}-phenyl]-6-(4-methoxyphenyl)-1,3,5-triazine, 2-hydroxy-4-methoxybenzophenone, hydroxymethoxybenzophenonesulfonic acid and a trihydrate thereof, sodium hydroxymethoxybenzophenone sulfonate, phenylbenzimidazolesulfonic acid, and 2,2'-methylenebis(6-(2H-benzotriazol-2-yl)-4-(1,1,3,3-tetramethylbutyl)phenol). A UVA absorber (such as diethylamino hydroxybenzoyl hexyl benzoate) and a UVB absorber (such as ethylhexyl methoxycinnamate) may be used together, and each may be arbitrarily combined.

Among them, one or at least two organic ultraviolet absorbers selected from ethylhexyl methoxycinnamate, diethylamino hydroxybenzoyl hexyl benzoate, octyl salicylate, polysilicone-15, t-butyl methoxydibenzoylmethane, oxybenzone, methylene bis-benzotriazolyl tetramethylbutylphenol, bis-ethylhexyloxyphenol methoxyphenyl triazine, and octocrylene are particularly preferred from the aspect of compatibility with oil materials.

When the oil material is added, components, the oil of which is not absorbed by component (A), or volatile oils are preferably selected. When a hydrocarbon oil, ester oil, or the like that is a sebum-like component is added, the amount of sebum absorbed may lower in some cases. When a non-volatile hydrocarbon oil or ester oil is added, the amount is preferably 100% or less, preferably 80% or less, and further preferably 50% or less, based on the amount of component (A), and most preferably, they are not added.

### (3) Powders other than afore-mentioned component (A)

The powders are not particularly limited as long as they have raw materials that may be added to ordinary cosmetics. Examples of the powders include pigments and silicone spherical powders. When the powders are added, the amount of the powders is not particularly limited but is preferably 0.1 % by mass to 90 % by mass, further preferably 1 % by mass to 35 % by mass of an entire cosmetic.

### - Coloring pigment

Examples of the coloring pigment include red iron oxide, yellow iron oxide, white titanium oxide, black iron oxide, red iron oxide, ultramarine, Prussian blue, manganese violet, cobalt violet, chromium hydroxide, chromium oxide, cobalt oxide, cobalt titanate, iron oxide-doped titanium oxide, iron titanate, fired (titanium/titanium oxide), lithium cobalt titanate, cobalt titanate, titanium nitride, iron hydroxide, inorganic brown pigments such as γ-iron oxide, inorganic yellow pigments such as yellow ochre, and colored pigments such as tar-base dyes in lake form and natural dyes in lake form, and any of them may be used.

Furthermore, the pigment according to the present invention may have been treated on the surface partially or entirely with an inorganic compound such as alumina, aluminum hydroxide, silica, or hydrous silica.

The coloring pigment may be hydrophobically treated. The hydrophobic treatment means performing surface treatment on the coloring pigment with a hydrophobic treating agent. The surface hydrophobic treating agent of the coloring pigment according to the present invention is not particularly limited as long as it may impart hydrophobicity. Examples of the treating agent include silicone treating agents, waxes, paraffins, organofluorine compounds such as perfluoroalkyl and phosphates, surfactants, amino acids such as N-acylglutamic acid, and metal soaps such as aluminum stearate and magnesium myristate.

Furthermore, the above-described surface hydrophobic treating agents may be used alone or in combination of two or more of them.

Specific examples of the coloring pigment with the surface hydrophobically treated include KTP-09 series, particularly KTP-09W, KTP-09R, KTP-09Y, KTP-09B, and the like (produced by Shin-Etsu Chemical Co., Ltd.).

### - Inorganic powder

Examples of the inorganic powders include microparticles of zirconium oxide, zinc oxide, cerium oxide, magnesium oxide, barium sulfate, calcium sulfate, magnesium sulfate, calcium carbonate, magnesium carbonate, talc, cleaved talc, mica, kaolin, sericite, muscovite, synthetic mica, phlogopite, trilithionite, biotite, lepidolite, silicic acid, silicon dioxide, fumed silica, hydrous silicon dioxide, aluminum silicate, magnesium silicate, aluminum magnesium silicate, calcium silicate, barium silicate, strontium silicate, tungstic acid metal salts, hydroxyapatite, vermiculite, hydrargilite, bentonite, montmorillonite, hectorite, zeolite, ceramic, calcium secondary phosphate, alumina, aluminum hydroxide, boron nitride, boron nitride, or glass. Further, examples of inorganic colored pearl pigments include pearl pigments such as titanium oxide-coated mica, bismuth oxychloride, titanium oxide-coated bismuth oxychloride, titanium oxide-coated talc, fish scales, and titanium oxide-coated colored mica. Examples of microparticulate metal oxides include one or at least two kinds selected from microparticulate titanium oxide, microparticulate iron-containing titanium oxide, microparticulate zinc oxide, microparticulate cerium oxide, and composites thereof. These metal oxides may be composite powders with other powders. An average primary particle diameter is preferably 1 nm or more and 200 nm or less, further preferably 120 nm or less. If the particle diameter is larger than the aforesaid upper limit, a defensive function against ultraviolet rays decreases, causing a white residue. The average primary particle diameter may be determined using transmission electron microscope photographs.

The above-described microparticulate metal oxides are not particularly limited even if they are unprocessed or processed with a known surface treatment used for the cosmetic products. Further, the microparticulate metal oxides may be dispersing elements that are preliminarily dispersed in volatile oil materials or ester oils. Specific examples of the dispersing elements include SPD series, particularly SPD-T5, SPD-T5L, SPD-T6, SPD-T7, SPD-Z5, SPD-Z5L, and the like (produced by Shin-Etsu Chemical Co., Ltd.).

### - Metal powder

Examples of metal powders include metal microparticles made of aluminum, copper, stainless steel, silver, and the like.

### - Organic powder

Examples of the organic powders include powders made of silicone, polyamide, polyacrylic acid-acrylate, polyester, polyethylene, polypropylene, polystyrene, styrene-acrylic acid copolymers, divinylbenzene-styrene copolymers, polyurethane, vinyl resins, urea resins, melamine resins, benzoguanamine, polymethylbenzoguanamine, tetrafluoroethylene, polymethyl methacrylate (such as polymethyl methacrylate), cellulose, silk, nylon, phenolic resins, epoxy resins, polycarbonate, and the like. Specific examples of those made of silicone include silicone resin particles (as specific examples, KMP-590, KMP-591, KMP-592, and the like produced by Shin-Etsu Chemical Co., Ltd.) and silicone resin-coated silicone rubber powders (as specific examples, KSP-100, KSP-101, KSP-102, KSP-105, KSP-300, KSP-411, KSP-441, and the like produced by Shin-Etsu Chemical Co., Ltd.), and they may preliminarily be dispersed in water or in oil. Metal soaps and the like are also included, and specific examples include powders of zinc stearate, aluminum stearate, calcium stearate, magnesium stearate, zinc myristate, magnesium myristate, zinc cetyl phosphate, calcium cetyl phosphate, zinc sodium cetyl phosphate, and the like. Organic dyes and the like are also included, and specific examples include synthetic dyes (tar dyes) such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207, and natural dyes such as carminic acid, laccaic acid, carthamin, brazilin, and crocin.

### - Inorganic/organic composite powder

Examples of inorganic/organic composite powders include composite powders in the form of inorganic particles with surfaces coated with organic particles by a publicly known and used method.

As the powders described above, those with particle surfaces treated may be used. Further, the surface treating agent preferably imparts hydrophobicity from the aspect of water resistance of cosmetics. The treating agent that imparts hydrophobicity is not particularly limited, and examples of the treating agent include silicone treating agents, waxes, paraffins, organofluorine compounds such as perfluoroalkyl and phosphates, surfactants, amino acids such as N-acylglutamic acid, and metal soaps such as aluminum stearate and magnesium myristate. More preferred are silicone treating agents. Examples include silanes or silylating agents such as caprylsilanes (AES-3083 produced by Shin-Etsu Chemical Co., Ltd.) or trimethoxysilyl dimethicone, silicone oils such as dimethylsilicones (KF-96A series produced by Shin-Etsu Chemical Co., Ltd.), methylhydrogen type polysiloxanes (KF-99P, KF-9901, and the like produced by Shin-Etsu Chemical Co., Ltd.), silicone branched silicone treating agents (KF-9908, KF-9909, and the like produced by Shin-Etsu Chemical Co., Ltd.), and acrylic silicones (KP-574 and KP-541 produced by Shin-Etsu Chemical Co., Ltd.). Furthermore, the above-described surface hydrophobic treating agents may be used alone or in combination of two or more of them.

### (4) Surfactant other than component (B)

Examples of the surfactant include nonionic, anionic, cationic, and ampholytic surfactants, and it is not particularly limited. Any of surfactants which are used in ordinary cosmetics may be used. Among these surfactants, preferred are partially crosslinked polyether-modified silicones, partially crosslinked polyglycerol-modified silicones, linear or branched polyoxyethylene-modified organopolysiloxanes, linear or branched polyoxyethylene polyoxypropylene-modified organopolysiloxanes, linear or branched polyoxyethylene/alkyl co-modified organopolysiloxanes, linear or branched polyoxyethylene polyoxypropylene/alkyl co-modified organopolysiloxanes, linear or branched polyglycerol-modified organopolysiloxanes, linear or branched polyglycerol/alkyl co-modified organopolysiloxanes, and linear or branched pyrrolidone-modified organopolysiloxanes. In these surfactants, the content of hydrophilic polyoxyethylene groups, polyoxyethylene polyoxypropylene groups, or polyglycerol residues is preferably 10 % by mass to 70 % by mass of the molecule. When a partially crosslinked polyether-modified silicone or partially crosslinked polyglycerol-modified silicone is used, in the compositions comprising the crosslinked organopolysiloxane and an oil material which is liquid at room temperature, it is preferred that the crosslinked organopolysiloxane swells to the liquid oil by containing an amount of the own weight or more of the liquid oil material. As the liquid oil material, liquid silicones, hydrocarbon oils, ester oils, natural vegetable and animal oils, semi-synthetic oils and fluorine-based oils, that described as an oil material of arbitrary components may be used. Examples of the liquid oil material include low viscosity silicones having a viscosity of 0.65 mm²/s to 100 mm²/s (at 25°C), hydrocarbon oils such as liquid paraffin, squalane, isododecane, and isohexadecane, glyceride oils such as trioctanoin, ester oils such as isotridecyl isononanoate, N-acylglutamate, and N-lauroyl sarcosinate, and natural vegetable and animal oils such as macadamia nut oil. Specific examples include KSG-210, KSG-240, KSG-310, KSG-320, KSG-330, KSG-340, KSG-320Z, KSG-350Z, KSG-710, KSG-810, KSG-820, KSG-830, KSG-840, KSG-820Z, and KSG-850Z produced by Shin-Etsu Chemical Co., Ltd. Specific examples of surfactants that are not crosslinked organopolysiloxanes include KF-6011, KF-6013, KF-6043, KF-6017, KF-6028, KF-6038, KF-6048, KF-6100, KF-6104, KF-6105, and KF-6106 produced by Shin-Etsu Chemical Co., Ltd. In any case, the amount of the surfactant is preferably 0.1 % by mass to 20 % by mass, based on an entire cosmetic. When the amount is 0.1% or more, sufficiently performing the functions of dispersion and emulsification is attained and when the amount is 20 % by mass or less, eliminates the possibility that the cosmetic has a greasy use feeling, those are preferable. Although not limited, the surfactant has an HLB of preferably 2 to 14.5 in order to maintain the water resistance of the cosmetics.

### (5) Crosslinked organopolysiloxane

The crosslinked organopolysiloxane is not particularly limited as long as it is used in ordinary cosmetic products. One kind may be used alone, or two or more kinds may be used in combination as necessary. Unlike the silicone powders described in the above (3) or component (A), the crosslinked organopolysiloxane does not have a spherical shape. Further, unlike the surfactant (4), the crosslinked organopolysiloxane is a compound that does not have a polyether or polyglycerol structure in the molecular structure. The crosslinked organopolysiloxane is an elastomer that has a structural viscosity by swelling with the oil material (2). Specific examples of the crosslinked organopolysiloxane include dimethicone/vinyl dimethicone crosspolymers, dimethicone/phenyl vinyl dimethicone crosspolymers, vinyl dimethicone/lauryl dimethicone crosspolymers, lauryl polydimethylsiloxyethyl dimethicone/bis-vinyldimethicone crosspolymers, which are defined as cosmetic product labeling names. These are commercially available as swollen materials comprising a liquid oil at room temperature, and specific examples include KSG-15, KSG-1510, KSG-16, KSG-1610, KSG-18A, KSG-19, KSG-41A, KSG-42A, KSG-43, KSG-44, KSG-042Z, KSG-045Z, and KSG-048Z produced by Shin-Etsu Chemical Co., Ltd. When this component is comprised, the amount is preferably 0.01 % by mass to 30 % by mass in a cosmetic as a solid content.

### (6) Film-forming agent

The film-forming agent is not particularly limited as long as it has a raw material that may be added to ordinary cosmetics. Specifically, examples of the film-forming agent includes latexes such as polyvinyl alcohol, polyvinyl pyrrolidone, polyvinyl acetate, and alkyl polyacrylate, cellulose derivatives such as dextrin, alkyl cellulose, and nitrocellulose, siliconized polysaccharide compounds such as trimethylsiloxysilylcarbamoyl pullulan, acrylic-silicone-based graft copolymers such as acrylates/dimethicone copolymers, silicone resins such as trimethylsiloxysilicate, silicone-based resins such as silicone-modified polynorbornene and fluorine-modified silicone resins, fluororesins, aromatic hydrocarbon resins, polymer emulsion resins, terpene-based resins, polybutene, polyisoprene, alkyd resins, polyvinyl pyrrolidone-modified polymers, rosin-modified resins, and polyurethane.

Among these, silicone-based film-forming agents are particularly preferred, and among them, although not limited, trimethylsiloxysilylcarbamoyl pullulan (dissolved in solvents to be available as TSPL-30-D5 and TSPL-30-ID from Shin-Etsu Chemical Co., Ltd. as commercial products), acrylates/dimethicone copolymers (dissolved in solvents to be available as KP-543, KP-545, KP-549, KP-550, KP-545L, and the like from Shin-Etsu Chemical Co., Ltd. as commercial products), trimethylsiloxysilicate (dissolved in solvents to be available as KF-7312J, X-21-5250, and the like from Shin-Etsu Chemical Co., Ltd. as commercial products), silicone-modified polynorbornene (dissolved in solvents to be available as NBN-30-ID and the like from Shin-Etsu Chemical Co., Ltd. as commercial products), and organosiloxane graft polyvinyl alcohol-based polymers may be used. As the film-forming agent, one or at least two kinds may be used. When this component is added, the amount is preferably 0.1 % by mass to 20 % by mass in a cosmetic.

### (7) Other Additives

Examples of the other additives include an oil-soluble gelling agent, water-soluble thickening agent, antiperspirant, preservative and antimicrobial, perfume, salt, antioxidant, pH adjuster, chelator, refrigerant, anti-inflammatory agent, skincare component (such as skin-brightening agent, cell activator, rough skin improver, blood circulation promoter, skin astringent, antiseborrheic agent), vitamin, amino acid, water-soluble polymer compound, fiber, and inclusion compound.

### - Oil-Soluble Gelling Agent

Examples of the oil-soluble gelling agent include metal soaps such as aluminum stearate, magnesium stearate, and zinc myristate; amino acid derivatives such as N-lauroyl-L-glutamic acid and α,γ-di-n-butylamine; dextrin fatty acid esters such as dextrin palmitic acid ester, dextrin stearic acid ester, and dextrin 2-ethylhexanoic acid/palmitic acid ester; sucrose fatty acid esters such as sucrose palmitic acid ester and sucrose stearic acid ester; fructo-oligosaccharide fatty acid esters such as fructo-oligosaccharide stearic acid ester and fructo-oligosaccharide 2-ethylhexanoic acid ester; benzylidene derivatives of sorbitol such as monobenzylidene sorbitol and dibenzylidene sorbitol; and organic-modified clay minerals of disteardimonium hectorite, stearalkonium hectorite, and hectorite.

### - Water-soluble thickening agent

Examples of the water-soluble thickening agent include plant polymers such as Arabia gum, tragacanth, galactan, carob gum, guar gum, karaya gum, carrageenan, pectin, agar, quince seed (marmelo), starch (such as rice, corn, potato, and wheat), algae colloid, trant gum, and locust bean gum; microbial polymers such as xanthan gum, dextran, succinoglucan, and pullulan; animal polymers such as collagen, casein, albumin, and gelatin; starch polymers such as carboxymethyl starch and methyl hydroxypropyl starch; cellulose polymers, such as methyl cellulose, ethyl cellulose, methyl hydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose, cationized cellulose, and cellulose powder; alginic acid polymers, such as sodium alginate and propylene glycol alginate ester; vinyl polymers such as polyvinyl methyl ether and carboxy vinyl polymer; polyoxyethylene polymers; polyoxyethylene polyoxypropylene copolymer polymers; acryl polymers such as sodium polyacrylate, polyethyl acrylate, polyacrylamide, and acryloyldimethyl taurate salt copolymer; other synthetic water-soluble polymers such as polyethyleneimine and cationic polymer; inorganic water-soluble polymers such as bentonite, aluminum magnesium silicate, montmorillonite, beidellite, nontronite, saponite, hectorite, and anhydrous silicic acid.

Among them, one or at least two water-soluble thickening agents selected from plant polymers, microbial polymers, animal polymers, starch polymers, cellulose polymers, alginic acid polymers, polyoxyethylene polyoxypropylene copolymer polymers, acryl polymers, and inorganic water-soluble polymers are preferably used.

### - Antiperspirant

Examples of the antiperspirant include aluminum hydroxyhalides such as chlorohydroxy aluminum and aluminum chlorohydroxy allantoinate; aluminum halides such as aluminum chloride; aluminum allantoinate, tannic acid, persimmon tannin, potassium aluminum sulfate, zinc oxide, zinc para-phenolsulfonate, burnt alum, aluminum zirconium tetrachlorohydrate, and aluminum zirconium trichlorohydrex glycine. In particular, as components that exhibit a high effect, an aluminum hydroxyhalide, an aluminum halide, and a complex or mixture thereof with zirconyl oxyhalide and zirconyl hydroxyhalide (for example, aluminum zirconium tetrachlorohydrate and aluminum zirconium trichlorohydrex glycine), and the like are preferred.

### - Preservative and Antimicrobial

Examples of the preservative and antimicrobial include para-oxybenzoate alkyl ester, benzoic acid, sodium benzoate, sorbic acid, potassium sorbate, phenoxy ethanol, imidazolidinyl urea, salicylic acid, isopropylmethylphenol, carbolic acid, p-chloro-m-cresol, hexachlorophene, benzalkonium chloride, chlorhexidine chloride, trichlorocarbanilide, iodopropynyl butylcarbamate, polylysine, photosensitizers, silver, and plant extracts.

### - Perfume

Examples of the perfume include natural perfumes and synthetic perfumes. Examples of the natural perfumes include vegetable perfumes separated from flowers, leaves, wood, pericarp, and the like, and animal perfumes such as musk and civet. Examples of the synthetic perfumes include hydrocarbons such as monoterpene; alcohols such as aliphatic alcohol and aromatic alcohol; aldehydes such as terpene aldehyde and aromatic aldehyde; ketones such as alicyclic ketone; esters such as terpene-based ester; lactones; phenols; oxides; nitrogen-containing compounds; and acetals.

### - Salt

Examples of the salt include inorganic salts, organic acid salts, amine salts, and amino acid salts. Examples of the inorganic salts include a sodium salt, potassium salt, magnesium salt, calcium salt, aluminum salt, zirconium salt, zinc salt, and the like of inorganic acids such as hydrochloric acid, sulfuric acid, carbonic acid, and nitric acid. Examples of the organic acid salts include salts of organic acids such as acetic acid, dehydroacetic acid, citric acid, malic acid, succinic acid, ascorbic acid, and stearic acid. Examples of the amine salts and the amino acid salts include salts of amines such as triethanolamine and salts of amino acids such as glutamic acid. In addition, as others, a salt of hyaluronic acid, chondroitin sulfuric acid, or the like, and further an acid-alkali neutralizing salt or the like used in preparation prescription may also be used.

### - Antioxidant

Examples of the antioxidant include, but are not particularly limited to, carotenoid, ascorbic acid and a salt thereof, ascorbyl stearate, tocophenol, tocophenol acetate, tocopherol, p-t-butylphenol, butylhydroxyanisol, dibutylhydroxytoluene, phytic acid, ferulic acid, thiotaurine, hypotaurine, sulfite, erythorbic acid and a salt thereof, chlorogenic acid, epicatechin, epigallocatechin, epigallocatechin gallate, apigenin, kaempferol, myricetin, and quercetin.

### - pH Adjuster

Examples of the pH adjuster include lactic acid, citric acid, glycolic acid, succinic acid, tartaric acid, dl-malic acid, potassium carbonate, sodium bicarbonate, and ammonium bicarbonate.

### - Chelator

Examples of the chelator include alanine, sodium edetate, sodium polyphosphate, sodium metaphosphate, and phosphoric acid.

### - Refrigerant

Examples of the refrigerant include L-menthol, camphor, and menthyl lactate.

### - Anti-inflammatory agent

Examples of the anti-inflammatory agent include allantoin, glycyrrhizinic acid and a salt thereof, glycyrrhetinic acid and stearyl glycyrrhetinate, tranexamic acid, and azulene.

### - Skincare component

Examples of the skincare component include skin-brightening agents such as placenta extract, arbutin, glutathione, and strawberry geranium extract; cell activators such as royal jelly, photosensitizer, cholesterol derivative, and calf blood extract; rough skin improvers; blood circulation promoters such as vanillylamide nonylate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-orizanol; skin astringents; and antiseborrheic agents such as sulfur and thianthrol.

### - Vitamin

Examples of the vitamin include vitamin A such as vitamin A oil, retinol, retinol acetate, and retinol palmitate; vitamin B including vitamin B₂ such as riboflavin, riboflavin butyrate, and flavin adenine nucleotide, vitamin B₆ such as pyridoxine hydrochloride, pyridoxine dioctanoate, and pyridoxine tripalmitate, vitamin B₁₂ and a derivative thereof, and vitamin B₁₅ and a derivative thereof; vitamin C such as L-ascorbic acid, L-ascorbic acid dipalmitate, sodium L-ascorbic acid-2-sulfate, and dipotassium L-ascorbic acid diphosphate; vitamin D such as ergocalciferol and cholecalciferol; vitamin E such as α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and amide nicotinate; pantothenic acids such as vitamin H, vitamin P, calcium pantothenate, D-pantothenyl alcohol, pantothenyl ethyl ether, and acetyl pantothenyl ethyl ether; and biotin.

### - Amino acid

Examples of the amino acid include glycine, valine, leucine, isoleucine, serine, threonine, phenylalanine, arginine, lysine, aspartic acid, glutamic acid, cystine, cysteine, methionine, and tryptophan.

### - Nucleic acid

Examples of a nucleic acid include deoxyribonucleic acid, and the like.

### - Hormone

Examples of a hormone include estradiol, ethenyl estradiol, and the like.

### - Inclusion compound

Examples of the inclusion compound include cyclodextrin, and the like.

The cosmetics described may be in any form of emulsion type or water type. When it is desired to impart an oily feeling, the emulsion form is selected. The emulsion form may be any form of oil-in-water (O/W) type emulsion, water-in-oil (W/O) type emulsion, oil-in-water-in-oil (O/W/O) type emulsion, or water-in-oil-in-water (W/O/W) type emulsion. When it is desired to obtain a fresh and youthful use feeling, an aqueous composition or powder composition may be selected. In both cases, satisfactory cosmetics may be obtained. Note that in the present invention, the "aqueous composition" means a composition in which an oil material is not purposely added. The "powder composition" is a composition in which a water phase is coated on microparticle powders or the powders are coated on the water phase, thereby obtaining a fresh and youthful use feeling by releasing the water phase immediately after applying a cosmetic over the skin even if it has powders in appearance. Among these, the aqueous composition with which an especially high sebum resistance may be expected is preferred.

The aqueous dispersion of silicone rubber particles of the present invention may be added to cosmetics by various kinds of methods. Examples include, in the case of the emulsion composition, preliminarily mixing the aqueous dispersion in a water phase, mixing it as a different phase from the water phase, or when adding the aqueous dispersion to an internal water phase, making droplets smaller to repel it to an external phase. The aqueous dispersion of the present invention may easily be added to the water phase.

While the cosmetics of the present invention are not particularly limited, for example, they are applicable to various products such as skin lotion, beauty essence, milky lotion, cream, hair care product, foundation, makeup base, sunscreen, concealer, cheek color, lipstick, gloss, balm, mascara, eye shadow, eyeliner, body make-up, deodorant, and manicure product. Among these, basic skin care products such as skin lotion, beauty essence, milky lotion, cream, makeup base, and deodorant are particularly preferred. The physical form of the cosmetics of the present invention may be selected from various physical forms such as liquid, cream, solid, paste, gel, mousse, souffle, clay, powder, and stick forms.

### EXAMPLES

The present invention will be explained below in further detail with reference to a series of the Examples and the Comparative Examples, though the present invention is in no way limited by these Examples.

In the examples, a dynamic viscosity is a value determined using a capillary viscosimeter at 25°C, and "%" representing a concentration and a content rate indicates "% by mass." An amount % of a long-chain monovalent hydrocarbon group means a percentage of the number of long-chain monovalent hydrocarbon group bonded to a silicon atom based on the total number of all substituents each bonded to a silicon atom.

### Example 1

In a glass beaker with a capacity of 1 liter, were put 400 g of organohydrogenpolysiloxane represented by formula (1) below and having 22.2% amount of the long-chain monovalent hydrocarbon group and a viscosity of 110 mm²/s, 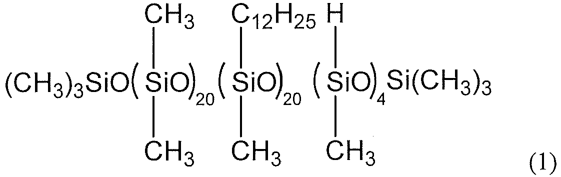 114 g of methylvinyl polysiloxane represented by formula (2) below and having no long-chain monovalent hydrocarbon group and a viscosity of 10 mm²/s, that has an amount such that the ratio of the number of vinyl groups in methylvinyl polysiloxane is 0.96, relative to one SiH group in the organohydrogenpolysiloxane, and 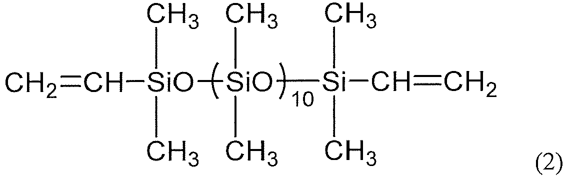 0.1 g of dl-α-tocopherol, and they were stirred and mixed at 2,000 rpm using a homomixer. To the resulting mixed liquid, 1.0 g of polyoxyethylene lauryl ether having 9 moles of added ethylene oxide and 190 g of water were added and stirred at 6,000 rpm using the homomixer, yielding an O/W type emulsion with an increased viscosity observed. The stirring was continued for another 15 minutes. Subsequently, 274 g of water was added while stirring at 2,000 rpm to obtain a uniform white emulsion.

This emulsion was transferred to a glass flask with a capacity of 1 liter equipped with a stirring device having an anchor-shaped stirring blade, and the temperature was adjusted to 15°C to 20°C. Then, 18 g of polyoxyethylene lauryl ether having 9 moles of added ethylene oxide was added to be dissolved in the emulsion by stirring for 30 minutes. Subsequently, a mixed solution comprising 2.4 g of isododecane solution of platinum-vinyl group-containing disiloxane complex (platinum content: 0.5%) and 1.0 g of polyoxyethylene lauryl ether having 9 moles of added ethylene oxide was added thereto under stirring, and they were stirred at the same temperature for 12 hours to obtain an aqueous dispersion of silicone rubber particles.

The silicone rubber particles were spherical in shape under observation with an optical microscope and had a volume average particle diameter of 12 µm as determined using a particle size distribution measurement instrument "Multisizer 3", trade name, produced by Beckman Coulter, Inc.

### Example 2

In a glass beaker with a capacity of 1 liter, were put 400 g of organohydrogenpolysiloxane represented by formula (3) below and having 10.5% of long-chain monovalent hydrocarbon groups and a viscosity of 130 mm²/s, 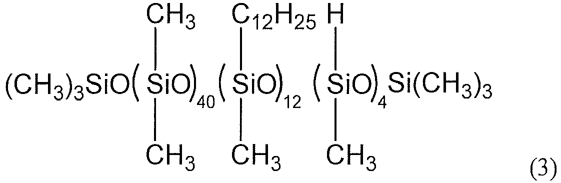 113 g of the methylvinyl polysiloxane represented by the formula (2), that has an amount such that the number vinyl groups in methylvinyl polysiloxane is 0.91, relative to one SiH group in the organohydrogenpolysiloxane and having no long-chain monovalent hydrocarbon group and a viscosity of 10 mm²/s, and 0.1 g of dl-α-tocopherol, and they were stirred and mixed at 2,000 rpm using a homomixer. To the resulting mixed liquid, 1.0 g of polyoxyethylene lauryl ether having 9 moles of added ethylene oxide and 190 g of water were added and stirred at 6,000 rpm using the homomixer, yielding an O/W type emulsion with an increased viscosity observed. The stirring was continued for another 15 minutes. Subsequently, 275 g of water was added while stirring at 2,000 rpm to obtain a uniform white emulsion.

This emulsion was transferred to a glass flask with a capacity of 1 liter equipped with a stirring device having an anchor-shaped stirring blade, and the temperature was adjusted to 15°C to 20°C. Then, 18 g of polyoxyethylene lauryl ether having 9 moles of added ethylene oxide was added to be dissolved in the emulsion by stirring for 30 minutes. Subsequently, a mixed solution comprising 2.4 g of isododecane solution of platinum-vinyl group-containing disiloxane complex (platinum content: 0.5%) and 1.0 g of polyoxyethylene lauryl ether having 9 moles of added ethylene oxide was added thereto under stirring, and they were stirred at the same temperature for 12 hours to obtain an aqueous dispersion of silicone rubber particles.

The silicone rubber particles were spherical in shape under observation with the optical microscope and had a volume average particle diameter of 12 µm as determined using the particle size distribution measurement instrument "Multisizer 3", trade name, produced by Beckman Coulter, Inc.

### Example 3

In a glass beaker with a capacity of 1 liter, were put 467 g of the organohydrogenpolysiloxane represented by the formula (1) and having 22.2% of long-chain monovalent hydrocarbon groups and a viscosity of 110 mm²/s, 133 g of the methylvinyl polysiloxane represented by the formula (2) and having no long-chain monovalent hydrocarbon group and a viscosity of 10 mm²/s, that has an amount such that the ratio of the number of vinyl groups in methylvinyl polysiloxane is 0.96, relative to one SiH group in the organohydrogenpolysiloxane, and 0.1 g of dl-α-tocopherol, and they were stirred and mixed at 2,000 rpm using a homomixer. To the resulting mixed liquid, 0.5 g of polyoxyethylene stearyl ether having 6 moles of added ethylene oxide, 1.6 g of polyoxyethylene stearyl ether having 20 moles of added ethylene oxide, 10 g of phenoxy ethanol, and 200 g of water were added and stirred at 6,000 rpm using the homomixer, yielding an O/W type emulsion with an increased viscosity observed. The stirring was continued for another 15 minutes. Subsequently, 164 g of water was added while stirring at 2,000 rpm to obtain a uniform white emulsion.

This emulsion was transferred to a glass flask with a capacity of 1 liter equipped with a stirring device having an anchor-shaped stirring blade, and the temperature was adjusted to 15°C to 20°C. Then, 5 g of polyoxyethylene stearyl ether having 6 moles of added ethylene oxide and 14 g of polyoxyethylene stearyl ether having 20 moles of added ethylene oxide were added to be dissolved in the emulsion by stirring for 30 minutes. Subsequently, a mixed solution comprising 2.5 g of isododecane solution of platinum-vinyl group-containing disiloxane complex (platinum content: 0.5%), 1.1 g of polyoxyethylene sorbitan tristearate having 20 moles of added ethylene oxide, and 1.1 g of polyoxyethylene sorbitan monostearate having 20 moles of added ethylene oxide was added thereto under stirring, and they were stirred at the same temperature for 12 hours to obtain an aqueous dispersion of silicone rubber particles.

The silicone rubber particles were spherical in shape under observation with the optical microscope and had a volume average particle diameter of 5 µm as determined using the particle size distribution measurement instrument "Multisizer 3", trade name, produced by Beckman Coulter, Inc.

### Comparative Example 1

In a glass beaker with a capacity of 1 liter, were put 19 g of methylhydrogenpolysiloxane represented by formula (4) below and having no long-chain monovalent hydrocarbon group and a viscosity of 30 mm²/s, 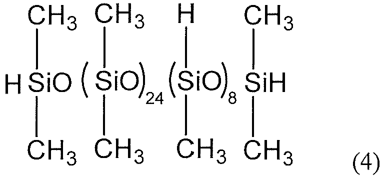 500 g of methylvinyl polysiloxane represented by formula (5) below and having no long-chain monovalent hydrocarbon group and a viscosity of 580 mm²/s, that has an amount such that the ratio of the number of vinyl groups in methylvinyl polysiloxane is 0.95, relative to one SiH group in organohydrogenpolysiloxane, and 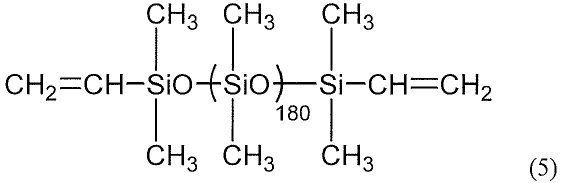 0.1 g of dl-α-tocopherol, and they were stirred and mixed at 2,000 rpm using a homomixer. To the resulting mixed liquid, 1.2 g of polyoxyethylene lauryl ether having 9 moles of added ethylene oxide and 100 g of water were added and stirred at 6,000 rpm using the homomixer, yielding an O/W type emulsion with an increased viscosity observed. The stirring was continued for another 15 minutes. Subsequently, 377 g of water was added while stirring at 2,000 rpm to obtain a uniform white emulsion. This emulsion was transferred to a glass flask with a capacity of 1 liter equipped with a stirring device having an anchor-shaped stirring blade, and the temperature was adjusted to 15°C to 20°C. Then, a mixed solution comprising 0.8 g of isododecane solution of platinum-vinyl group-containing disiloxane complex (platinum content: 0.5%) and 1.8 g of polyoxyethylene lauryl ether having 9 moles of added ethylene oxide was added thereto under stirring, and they were stirred at the same temperature for 12 hours to obtain an aqueous dispersion of silicone rubber particles.

The silicone rubber particles were spherical in shape under observation with the optical microscope and had a volume average particle diameter of 12 µm as determined using the "Multisizer 3."

### [Determination of hardness of silicone rubber particles]

Each of the organohydrogenpolysiloxanes, methylvinyl polysiloxanes, and isododecane solutions of platinum-vinyl group-containing disiloxane complex (platinum content: 0.5%) used in the Examples 1 to 3 and Comparative Example was mixed in each blend ratio described in the Examples 1 to 3 and Comparative Example, and the mixture was poured into an aluminum petri dish so as to have a thickness of 10 mm. After being allowed to stand at 25°C for 24 hours, the mixture was heated in a thermostatic oven at 50°C for 1 hour to obtain non-sticky silicone rubber. The hardness of the silicone rubber was determined using an Asker C hardness meter prescribed in SRIS 0101. The results are as shown in Table 1.

### [Determination of oil absorption amount of silicone rubber particles]

Each of the organohydrogenpolysiloxanes, methylvinyl polysiloxanes, and isododecane solutions of platinum-vinyl group-containing disiloxane complex (platinum content: 0.5%) used in the Examples 1 to 3 and Comparative Example was mixed in each blend ratio described in the Examples 1 to 3 and Comparative Example, and the mixture was poured into a Teflon (registered trademark) tray (80 mm×130 mm) so as to have a thickness of about 1 mm. After being allowed to stand at 25°C for 24 hours, the mixture was heated in a thermostatic oven at 50°C for 1 hour to obtain a sheet of silicone rubber. The obtained sheet was cut into square pieces with a length along one side of approximately 30 mm to obtain test pieces. After the mass of the test pieces were determined, the test pieces were dipped in respective oily substances shown in Table 1 for 24 hours. This caused the test pieces to absorb the oily substances and swell. The test pieces were removed from the oily substances and had the oily substances on the surfaces wiped off with tissues and, then, the mass of the test pieces was determined. The amounts of the oily substances absorbed (the oil absorption amount) by the sheets of silicone rubber shown in Table 1 are values per gram of the silicone rubber.

**[Table 1]**

| | | Example 1 | Example 2 | Example 3 | Comparative Example 1 |
|---|---|---|---|---|---|
| Oil absorption amount per gram of silicone rubber (g) | Squalane | 1.6 | 0.7 | 1.6 | 0.1 or less |
| | Ethylhexyl palmitate | 1.5 | 0.7 | 1.5 | 0.2 |
| | Liquid paraffin | 0.7 | 0.4 | 0.7 | 0.1 or less |
| | Isocetyl myristate | 1.1 | 0.6 | 1.1 | 0.1 or less |
| | Isocetyl isostearate | 1 | 0.5 | 1 | 0.1 or less |
| Hardness of silicone rubber | | 44 | 53 | 44 | 55 |

### Examples 4 to 6 and Comparative Examples 2 to 5

Each components were mixed in a composition shown in Table 2 below to prepare a gel milky lotion.

**[Table 2]**

| Component | | Example | | | Comparative Example | | | |
|---|---|---|---|---|---|---|---|---|
| | | 4 | 5 | 6 | 2 | 3 | 4 | 5 |
| 1 | Aqueous dispersion of Example 1 (51.4% ^{Note 3}) | 9.7 | | | | | | |
| | Aqueous dispersion of Example 2 (51.3% ^{Note 3}) | | 9.7 | | | | | |
| | Aqueous dispersion of Example 3 (60% ^{Note 3}) | | | 8.3 | | | | |
| | Aqueous dispersion of Comparative Example 1 (51.9% ^{Note 3}) | | | | | 9.6 | | |
| 2 | KSP-441 (Note 1) | | | | | | 5.0 | |
| 3 | KSG-42A (Note 2) | | | | | | | 25.0 |
| 4 | Polyoxyethylene lauryl ether (moles of added EO is 9 moles) | | | | | | 0.2 | 0.2 |
| 5 | Ethanol | 3 | 3 | 3 | 3 | 3 | 3 | 3 |
| 6 | Glycerin | 5 | 5 | 5 | 5 | 5 | 5 | 5 |
| 7 | BG | 5 | 5 | 5 | 5 | 5 | 5 | 5 |
| 8 | Carbomer (2% aqueous solution) | 12 | 12 | 12 | 12 | 12 | 12 | 12 |
| 9 | EDTA-2Na (10% aqueous solution) | 0.5 | 0.5 | 0.5 | 0.5 | 0.5 | 0.5 | 0.5 |
| 10 | Arginine (10% aqueous solution) | 2 | 2 | 2 | 2 | 2 | 2 | 2 |
| 11 | Water | Remai ning amount | Remai ning amount | Remai ning amount | Remai ning amount | Remai ning amount | Remai ning amount | Remai ning amount |
| | Total | 100.0 | 100.0 | 100.0 | 100.0 | 100.0 | 100.0 | 100.0 |
| | Powder amount | 5.0 | 5.0 | 5.0 | 0.0 | 5.0 | 5.0 | 5.0 |

| | | | | | | | | |
|---|---|---|---|---|---|---|---|---|
| (Note 1) Polysilicone-22 (labeling name) (INCI: Polysilicone-22) produced by Shin-Etsu Chemical Co., Ltd. (Note 2) Mixture of 20% of vinyl dimethicone/lauryl dimethicone crosspolymer (labeling name) (INCI: Vinyl Dimethicone/Lauryl Dimethicone Crosspolymer) and 80% of isododecane (labeling name) (INCI: Isododecane), produced by Shin-Etsu Chemical Co., Ltd. (Note 3) The value of % in parentheses is concentration of silicone rubber particles in the aqueous dispersion. | | | | | | | | |

### (Preparation Method)

Components 5 to 11 were mixed so as to be uniform. Components 1 to 4, which had been uniformly mixed, were gently added thereto, and they were stirred. This mixture was filled in a predetermined vessel to obtain a gel milky lotion.

### [Usability Evaluation]

The obtained gel milky lotions were evaluated by 10 specialized female panelists based on criteria below regarding the use feeling (no stickiness), finish (whether to blur pores and wrinkles), adherence (adhesion to the skin), sebum resistance (no shininess over time after application), and dispersibility (whether to generate lumps when manually stirring and mixing) to take the average of the evaluation results.

**[Table 3]**

| Item | Use feeling | Finish | Adherence | Sebum resistance | Dispersibility |
|---|---|---|---|---|---|
| 5 points | Good | Good | Good | Good | Good |
| 4 points | Slightly good | Slightly good | Slightly good | Slightly good | Slightly good |
| 3 points | Fair | Fair | Fair | Fair | Fair |
| 2 points | Slightly poor | Slightly poor | Slightly poor | Slightly poor | Slightly poor |
| 1 point | Poor | Poor | Poor | Poor | Poor |

Based on the average score of the obtained evaluation results, the results based on the following are shown in Table 5.
Excellent: Average score of 4.0 points or more
Good: Average score of 3.0 points or more and less than 4.0 points
Fair: Average score of 2.0 points or more and less than 3.0 points
Poor: Average score of less than 2.0 points

**[Table 4]**

| Property evaluation | Examples | | | Comparative Examples | | | |
|---|---|---|---|---|---|---|---|
| | 4 | 5 | 6 | 2 | 3 | 4 | 5 |
| Use feeling | Good | Good | Excellent | Poor | Good | Excellent | Fair |
| Finish | Excellent | Good | Excellent | Poor | Fair | Excellent | Fair |
| Adherence | Excellent | Good | Excellent | Poor | Fair | Poor | Poor |
| Sebum resistance | Excellent | Good | Excellent | Poor | Fair | Excellent | Excellent |
| Dispersibility | Excellent | Excellent | Excellent | - | Excellent | Poor | Poor |

As described in the Table 4, the cosmetics of the present invention were significantly excellent in the use feeling, finish, adherence, and sebum resistance compared with those of Comparative Examples 2 to 5. Further, even if elastomers having sebum resistance of Comparative Examples 4 and 5 were used, the dispersibility to the water phase was not preferable, and the adherence was not preferable because they could not beautifully be applied on the skin.

The following shows examples of the cosmetics. In the following, an evaluation was performed based on the same criteria as those described above.

### [Example 7]

### O/W Gel

### <Preparation of Cosmetic>

A: Components 2 to 12 were uniformly mixed.
B: Component 1 was added to the mixture of A, and they were emulsified to obtain an O/W gel.

| | Component | Mass% |
|---|---|---|
| 1. | KF-7312L (Note 1) | 2 |
| 2. | Aqueous dispersion of Example 3 (60%) | 5 |
| 3. | Sorbitol | 3 |
| 4. | Glycerin | 10 |
| 5. | BG | 5 |
| 6. | KF-6100 (Note 2) | 0.5 |
| 7. | PVP | 0.1 |
| 8. | Carbomer (2% aqueous solution) | 10 |
| 9. | Acrylates/C10-30 alkyl acrylate crosspolymer (2% aqueous solution) | 15 |
| 10. | EDTA-2Na (10% aqueous solution) | 0.5 |
| 11. | Sodium hydroxide (10% aqueous solution) | 0.6 |
| 12. | Water | 48.3 |
| | Total | 100 |

| | | |
|---|---|---|
| (Note 1) Trimethylsiloxysilicate (labeling name) (INCI: Trimethylsiloxysilicater) produced by Shin-Etsu Chemical Co., Ltd. (Note 2) Polyglyceryl-3 disiloxane dimethicone (labeling name) (INCI: Polyglyceryl-3 Disiloxane Dimethicone) produced by Shin-Etsu Chemical Co., Ltd. | | |

The obtained O/W gel was excellent in the use feeling, finish, adherence, and sebum resistance.

### [Example 8]

### O/W Base

### <Preparation of Cosmetic>

A: Components 1 to 7 were uniformly mixed at 90°C.
B: Components 8 to 13 were uniformly mixed at 85°C.
C: The mixture of B was added to the mixture of A, and they were emulsified and cooled. Afterwards, components 14 and 15 were each added thereto, and they were uniformly mixed to obtain a base.

| | Component | Mass% |
|---|---|---|
| 1. | BG | 6 |
| 2. | PEG-20 glyceryl isostearate | 1.2 |
| 3. | KF-96A-100cs | 2 |
| 4. | KF-96A-300cs | 1 |
| 5. | Behenyl alcohol | 0.5 |
| 6. | KSP-300 (Note 1) | 1 |
| 7. | Stearyl glycyrrhizinate | 0.1 |
| 8. | Diglycerine | 2 |
| 9. | Ethylhexylglycerin | 0.1 |
| 10. | BG | 8 |
| 11. | Sorbitol | 3 |
| 12. | Acrylates/C10-30 alkyl acrylate crosspolymer | 0.2 |
| 13. | Water | 54.5 |
| 14. | Aqueous dispersion of Example 3 (60%) | 20 |
| 15. | Potassium hydroxide (10% aqueous solution) | 0.5 |
| | Total | 100 |

| | | |
|---|---|---|
| (Note 1) Diphenyl dimethicone/vinyl diphenyl dimethicone/silsesquioxane crosspolymer (labeling name) (INCI: Diphenyl Dimethicone/Vinyl Diphenyl Dimethicone/Silsesquioxane Crosspolymer) produced by Shin-Etsu Chemical Co., Ltd. | | |

The obtained O/W base was excellent in the use feeling, finish, adherence, and sebum resistance and exhibited excellent persistence in overlaid makeup cosmetics.

### [Example 9]

### O/W Sunscreen Cream

### <Preparation of Cosmetic>

A: Components 1 to 6 were heated to 85°C and uniformly mixed.
B: Components 8 to 15 were heated to 85°C and uniformly mixed.
C: The mixture of B was added to the mixture of A, and they were emulsified and cooled. Afterwards, component 7 was added thereto, and they were uniformly mixed to obtain a sunscreen.

| | Component | Mass% |
|---|---|---|
| 1. | Hydroxyethyl cellulose | 0.1 |
| 2. | Ethanol | 10 |
| 3. | 1,3-butylene glycol | 6 |
| 4. | Allantoin | 0.1 |
| 5. | SIMULGEL NS (Note 1) | 2.5 |
| 6. | Water | Remaining amount |
| 7. | Aqueous dispersion of Example 3 (60%) | 10 |
| 8. | KF-56A (Note 2) | 3 |
| 9. | Cetanol | 2 |
| 10. | Ethylhexyl methoxycinnamate | 7.5 |
| 11. | Diethylamino hydroxybenzoyl hexyl benzoate | 2 |
| 12. | Ethylhexyl salicylate | 5 |
| 13. | PEG-60 hydrogenated castor oil | 1 |
| 14. | KF-6043 (Note 3) | 0.5 |
| 15. | Tocopherol | 0.05 |
| | Total | 100 |

| | | |
|---|---|---|
| (Note 1) Hydroxyethyl acrylate/sodium acryloyldimethyl taurate copolymer composition produced by SEPPIC (Note 2) Diphenylsiloxy phenyl trimethicone (labeling name) (INCI: Diphenylsiloxy Phenyl Trimethicone) produced by Shin-Etsu Chemical Co., Ltd. (Note 3) PEG-10 dimethicone (labeling name) (INCI: PEG-10 Dimethicone) produced by Shin-Etsu Chemical Co., Ltd. | | |

The obtained O/W sunscreen cream was excellent in the use feeling, finish, adherence, and sebum resistance.

### [Example 10]

### O/W Sunscreen Cream

### <Preparation of Cosmetic>

A: Components 1 to 6 were heated to 85°C and uniformly mixed.
B: Components 8 to 14 were heated to 85°C and uniformly mixed.
C: The mixture of B was added to the mixture of A, and they were emulsified and cooled. Afterwards, component 7 was added thereto, and they were uniformly mixed to obtain a sunscreen.

| | Component | Mass% |
|---|---|---|
| 1. | Hydroxyethyl cellulose | 0.1 |
| 2. | Ethanol | 5 |
| 3. | BG | 6 |
| 4. | Allantoin | 0.1 |
| 5. | SIMULGEL NS (Note 1) | 2.5 |
| 6. | Water | Remaining amount |
| 7. | Aqueous dispersion of Example 3 (60%) | 5 |
| 8. | TMF-1.5 (Note 2) | 3 |
| 9. | Cetanol | 2 |
| 10. | SPD-Z5L (Note 3) | 20 |
| 11. | Isotridecyl isononanoate | 3 |
| 12. | PEG-60 hydrogenated castor oil | 1 |
| 13. | PEG-20 glyceryl isostearate | 0.5 |
| 14. | Tocopherol | 0.05 |
| | Total | 100 |

| | | |
|---|---|---|
| (Note 1) Hydroxyethyl acrylate/sodium acryloyldimethyl taurate copolymer composition produced by SEPPIC (Note 2) Methyl trimethicone (labeling name) (INCI: Methyl Trimethicone) produced by Shin-Etsu Chemical Co., Ltd. (Note 3) Dispersion of 60% zinc oxide (labeling name) (INCI: Zinc Oxide) produced by Shin-Etsu Chemical Co., Ltd. | | |

The obtained O/W sunscreen cream was excellent in the use feeling, finish, adherence, and sebum resistance.

### [Example 11]

### W/O/W Gel

### <Preparation of Cosmetic>

A: Components 1 and 2 were uniformly mixed.
B: Components 3 to 7 were uniformly mixed.
C: Components 8 to 13 were uniformly mixed.
D: The mixture of B was added to the mixture of A, and they were emulsified.
E: The resultant of D was added to the mixture of C, and they were emulsified to obtain a W/O/W gel.

| | Component | Mass% |
|---|---|---|
| 1. | KSG-210 (Note 1) | 1.5 |
| 2. | KF-96A-6cs | 5 |
| 3. | BG | 5 |
| 4. | Purified water | 35 |
| 5. | Magnesium ascorbyl phosphate | 3 |
| 6. | Sodium metabisulfite | 0.01 |
| 7. | Sodium hydroxide | 0.02 |
| 8. | Glycerin | 10 |
| 9. | Acrylates/C10-30 alkyl acrylate crosspolymer | |
| | (2% aqueous solution) | 15 |
| 10. | EDTA-2Na (10% aqueous solution) | 0.5 |
| 11. | Sodium hydroxide (10% aqueous solution) | 0.6 |
| 12. | Aqueous dispersion of Example 3 (60%) | 2 |
| 13. | Purified water | 22.37 |
| | Total | 100 |

| | | |
|---|---|---|
| (Note 1) 25% dimethicone/PEG-10/15 crosspolymer (labeling name) (INCI: Dimethicone/(PEG-10/15) crosspolymer) produced by Shin-Etsu Chemical Co., Ltd. | | |

The obtained W/O/W gel was excellent in the use feeling, finish, adherence, and sebum resistance.

### [Example 12]

### W/O Eye Cream

### <Preparation of Cosmetic>

A: Components 1 to 7 were uniformly mixed.
B: Components 8 to 14 were uniformly mixed.
C: The mixture of B was added to the mixture of A, and they were emulsified to obtain an eye cream.

| | Component | Mass% |
|---|---|---|
| 1. | KSG-380Z (Note 1) | 3 |
| 2. | KSG-048Z (Note 2) | 3 |
| 3. | KF-6048 (Note 3) | 0.6 |
| 4. | Squalane | 3 |
| 5. | Ethylhexyl palmitate | 6 |
| 6. | Dimethicone (2cs) | 5 |
| 7. | KSP-105 (Note 4) | 2 |
| 8. | Sodium hyaluronate | 0.1 |
| 9. | Erythritol | 5 |
| 10. | Methyl gluceth-10 | 3 |
| 11. | Aqueous dispersion of Example 3 (60%) | 5 |
| 12. | Pentylene glycol | 3 |
| 13. | Sodium chloride | 0.5 |
| 14. | Water | Remaining amount |
| | Total | 100 |

| | | |
|---|---|---|
| (Note 1) Mixture of 30% of (PEG-15/lauryl polydimethylsiloxyethyl dimethicone crosspolymer (labeling name) (INCI: PEG-15/Lauryl Polydimethylsiloxyethyl Dimethicone Crosspolymer) and 70% of dimethicone (labeling name) (INCI: Dimethicone), produced by Shin-Etsu Chemical Co., Ltd. (Note 2) Mixture of 20% of (lauryl polydimethylsiloxyethyl dimethicone/bis-vinyldimethicone crosspolymer (labeling name) (Lauryl Polydimethylsiloxyethyl Dimethicone/Bis-Vinyldimethicone Crosspolymer) and 80% of dimethicone (labeling name) (INCI: Dimethicone), produced by Shin-Etsu Chemical Co., Ltd. (Note 3) Cetyl PEG/PPG-10/1 dimethicone (labeling name) (INCI: Cetyl PEG/PPG-10/1 Dimethicone), produced by Shin-Etsu Chemical Co., Ltd. (Note 4) Vinyl dimethicone/methicone silsesquioxane crosspolymer (labeling name) (INCI: Vinyl Dimethicone/Methicone Silsesquioxane Crosspolymer), produced by Shin-Etsu Chemical Co., Ltd. | | |

The obtained W/O eye cream was excellent in the use feeling, finish, adherence, and sebum resistance.

### [Example 13]

### W/O Eye Cream

### <Preparation of Cosmetic>

A: Components 1 to 7 were uniformly mixed.
B: Components 8 to 14 were uniformly mixed.
C: The mixture of B was added to the mixture of A, and they were emulsified to obtain an eye cream.

| | Component | Mass% |
|---|---|---|
| 1. | KSG-820 (Note 1) | 4 |
| 2. | KSG-42A (Note 2) | 3 |
| 3. | KF-6105 (Note 3) | 2 |
| 4. | Jojoba seed oil | 4 |
| 5. | Mealowfoam oil | 6 |
| 6. | Shea butter | 1 |
| 7. | KP-550 (Note 4) | 2 |
| 8. | Sodium hyaluronate | 0.1 |
| 9. | Xylitol | 3 |
| 10. | PCA-Na | 2 |
| 11. | Aqueous dispersion of Example 3 (60%) | 5 |
| 12. | Pentylene glycol | 3 |
| 13. | Sodium chloride | 0.5 |
| 14. | Water | Remaining amount |
| | Total | 100 |

| | | |
|---|---|---|
| (Note 1) Mixture of 25% of (lauryl dimethicone/polyglycerin-3 crosspolymer (labeling name) (INCI: Lauryl Dimethicone/Polyglycerin-3 Crosspolymer) and 75% of isododecane (labeling name) (INCI: Isododecane), produced by Shin-Etsu Chemical Co., Ltd. (Note 2) Mixture of 20% of vinyl dimethicone/lauryl dimethicone crosspolymer (labeling name) (INCI: Vinyl Dimethicone/Lauryl Dimethicone Crosspolymer) and 80% isododecane (labeling name) (INCI: Isododecane), produced by Shin-Etsu Chemical Co., Ltd. (Note 3) Lauryl polyglyceryl-3 polydimethylsiloxyethyl dimethicone (labeling name) (INCI: Lauryl Polyglyceryl-3 Polydimethylsiloxyethyl Dimethicone), produced by Shin-Etsu Chemical Co., Ltd. (Note 4) Acrylates/dimethicone copolymer (labeling name) (INCI: Acrylates/Dimethicone Copolymer), produced by Shin-Etsu Chemical Co., Ltd. | | |

The obtained W/O eye cream was excellent in the use feeling, finish, adherence, and sebum resistance.

### [Example 14]

### Skin Lotion

### <Preparation of Cosmetic>

A: Components 1 to 9 were uniformly mixed to obtain a skin lotion.

| | Component | Mass% |
|---|---|---|
| 1. | Aqueous dispersion of Example 3 (60%) | 10 |
| 2. | Glycerin | 5 |
| 3. | Ethanol | 45 |
| 4. | Menthol | 0.3 |
| 5. | Polysorbate 20 | 0.3 |
| 6. | Perfume | 0.05 |
| 7. | Citric acid | 0.02 |
| 8. | Sodium citrate | 0.016 |
| 9. | Purified water | 39.314 |
| | Total | 100 |

The obtained skin lotion was excellent in the use feeling, finish, adherence, and sebum resistance. Furthermore, makeup coming off was improved by spraying the obtained skin lotion in a mist state or applying it to a cotton pad to press over the makeup.

### [Example 15]

### Beauty Essence

### <Preparation of Cosmetic>

A: Components 1 to 15 were uniformly mixed to obtain a beauty essence.

| | Component | Mass% |
|---|---|---|
| 1. | Aqueous dispersion of Example 3 (60%) | 5 |
| 2. | BG | 8 |
| 3. | Glycerin | 10 |
| 4. | Maltitol | 2 |
| 5. | Diglycerine | 2 |
| 6. | Pentylene glycol | 2 |
| 7. | Ethylhexylglycerin | 0.1 |
| 8. | Betaine | 0.5 |
| 9. | Xanthan gum | 0.1 |
| 10. | Sodium hyaluronate | 0.1 |
| 11. | Dipotassium glycyrrhizate | 0.2 |
| 12. | Arbutin | 3 |
| 13. | Sodium metabisulfite | 0.02 |
| 14. | Pentasodium pentetate | 0.02 |
| 15. | Purified water | Remaining amount |
| | Total | 100 |

The obtained beauty essence was excellent in the use feeling, finish, adherence, and sebum resistance.

### [Example 16]

### Out-Bath Treatment

### <Preparation of Cosmetic>

A: Components 1 to 7 were uniformly mixed.
B: Components 8 to 14 were uniformly mixed.
C: The mixture of B was added to the mixture of A, and they were emulsified to obtain an eye cream.

| | Component | Mass% |
|---|---|---|
| 1. | KSG-710 (Note 1) | 4 |
| 2. | KSG-19 (Note 2) | 3 |
| 3. | KF-6038 (Note 3) | 2 |
| 4. | KF-96L-2cs | 6 |
| 5. | Isododecane | 4 |
| 6. | KF-56A (Note 4) | 2 |
| 7. | KF-8020 (Note 5) | 0.5 |
| 8. | BG | 10 |
| 9. | Polyquaternium-51 | 0.5 |
| 10. | Sodium citrate | 0.2 |
| 11. | Aqueous dispersion of Example 3 (60%) | 5 |
| 12. | Methylparaben | 0.2 |
| 13. | Sodium chloride | 0.5 |
| 14. | Water | Remaining amount |
| | Total | 100 |

| | | |
|---|---|---|
| (Note 1) Mixture of 25% of dimethicone/polyglycerin-3 crosspolymer (labeling name) (INCI: Dimethicone/Polyglycerin-3 Crosspolymer) and 75% of dimethicone (labeling name) (INCI: Dimethicone), produced by Shin-Etsu Chemical Co., Ltd. (Note 2) Mixture of 15% of dimethicone/vinyl dimethicone crosspolymer (labeling name) (INCI: Dimethicone/Vinyl Dimethicone Crosspolymer) and 85% of dimethicone (labeling name) (INCI: Dimethicone), produced by Shin-Etsu Chemical Co., Ltd. (Note 3) Lauryl PEG-9 polydimethylsiloxyethyl dimethicone (labeling name) (INCI: Lauryl PEG-9 Polydimethylsiloxyethyl Dimethicone), produced by Shin-Etsu Chemical Co., Ltd. (Note 4) Diphenylsiloxy phenyl trimethicone (labeling name) (INCI: Diphenylsiloxy Phenyl Trimethicone), produced by Shin-Etsu Chemical Co., Ltd. (Note 5) Aminopropyl dimethicone (labeling name) (INCI: Aminopropyl Dimethicone), produced by Shin-Etsu Chemical Co., Ltd. | | |

The out-bath treatment was excellent in the use feeling, finish, adherence, and sebum resistance.

### [Example 17]

### Shake Type Foundation

### <Preparation of Cosmetic>

A: Components 1 to 14 were uniformly mixed with a homomixer.
B: Components 15 to 18 were uniformly mixed.
C: The mixture of B was added to the mixture of A, and they were emulsified to obtain a shake type foundation.

| | Component | Mass% |
|---|---|---|
| 1. | KSG-18A (Note 1) | 4 |
| 2. | KSG-15 (Note 2) | 3 |
| 3. | KF-6028 (Note 3) | 2.5 |
| 4. | Cyclopentasiloxane | Remaining amount |
| 5. | Ethylhexyl methoxycinnamate | 7.4 |
| 6. | Disteardimonium hectorite | 1 |
| 7. | KMP-590 (Note 4) | 8 |
| 8. | KF-6106 (Note 5) | 1.5 |
| 9. | KTP-09W (Note 6) | 10 |
| 10. | KTP-09Y (Note 7) | 1.2 |
| 11. | KTP-09R (Note 7) | 0.7 |
| 12. | KTP-09B (Note 7) | 0.1 |
| 13. | Alkylsilane-treated microparticulate zinc oxide | 15 |
| 14. | Metal soap-treated microparticulate titanium oxide | 5 |
| 15. | BG | 5 |
| 16. | Magnesium sulfate | 0.5 |
| 17. | Phenoxy ethanol | 0.3 |
| 18. | Aqueous dispersion of Example 3 (60%) | 15 |
| | Total | 100 |

| | | |
|---|---|---|
| (Note 1) Mixture of 15% of dimethicone/phenyl vinyl dimethicone crosspolymer (labeling name) (INCI: Dimethicone/Phenyl Vinyl Dimethicone Crosspolymer) and 85% of diphenylsiloxy phenyl trimethicone (labeling name) (INCI: Diphenylsiloxy), produced by Shin-Etsu Chemical Co., Ltd. (Note 2) Mixture of 7% of dimethicone/vinyl dimethicone crosspolymer (labeling name) (INCI: Dimethicone/Vinyl Dimethicone Crosspolymer) and 93% of cyclopentasiloxane (labeling name) (INCI: Cyclopentasiloxane), produced by Shin-Etsu Chemical Co., Ltd. (Note 3) PEG-9 polydimethylsiloxyethyl dimethicone (labeling name) (INCI: PEG-9 Polydimethylsiloxyethyl Dimethicone), produced by Shin-Etsu Chemical Co., Ltd. (Note 4) Polymethylsilsesquioxane (labeling name) (INCI: Polymethylsilsesquioxane) produced by Shin-Etsu Chemical Co., Ltd. (Note 5) Polyglyceryl-3 polydimethylsiloxyethyl dimethicone (labeling name) (INCI: Polyglyceryl-3 Polydimethylsiloxyethyl Dimethicone) produced by Shin-Etsu Chemical Co., Ltd. (Note 6) Titanium oxide treated with triethoxysilylethyl polydimethylsiloxyethyl hexyl dimethicone (labeling name) (INCI: Triethoxysilylethyl Polydimethylsiloxyethyl Hexyl Dimethicone) produced by Shin-Etsu Chemical Co., Ltd. (Note 7) Iron oxide treated with triethoxysilylethyl polydimethylsiloxyethyl hexyl dimethicone (labeling name) (INCI: Triethoxysilylethyl Polydimethylsiloxyethyl Hexyl Dimethicone) produced by Shin-Etsu Chemical Co., Ltd. | | |

The obtained shake type foundation was excellent in the use feeling, finish, adherence, and sebum resistance, and exhibited favorable makeup lasting quality.

### [Example 18]

### Mascara

### <Preparation of Cosmetic>

A: Components 1 to 11 were uniformly mixed at 90°C, and then cooled to 70°C.
B: Components 12 to 14 were uniformly mixed at 60°C.
C: The mixture of B was added to the mixture of A, and they were emulsified and cooled to obtain a mascara.

| | Component | Mass% |
|---|---|---|
| 1. | TSPL-30-ID (Note 1) | 10 |
| 2. | Isododecane | Remaining amount |
| 3. | Dextrin palmitate | 2 |
| 4. | Ceresine | 7 |
| 5. | Paraffin wax | 5 |
| 6. | Disteardimonium hectorite | 5 |
| 7. | KTP-09B (Note 2) | 5 |
| 8. | Silicone-treated (Note 3) talc | 5 |
| 9. | KSP-300 (Note 4) | 5 |
| 10. | KF-6028 (Note 5) | 1.2 |
| 11. | Propylene carbonate | 1.6 |
| 12. | Phenoxy ethanol | 0.2 |
| 13. | BG | 2 |
| 14. | Aqueous dispersion of Example 3 (60%) | 10 |
| | Total | 100 |

| | | |
|---|---|---|
| (Note 1) Trimethylsiloxysilylcarbamoyl pullulan (labeling name) (INCI: Trimethylsiloxysilylcarbamoyl Pullulan) produced by Shin-Etsu Chemical Co., Ltd. (Note 2) Iron oxide treated with triethoxysilylethyl polydimethylsiloxyethyl hexyl dimethicone (labeling name) (INCI: Triethoxysilylethyl Polydimethylsiloxyethyl Hexyl Dimethicone) produced by Shin-Etsu Chemical Co., Ltd. (Note 3) Triethoxysilylethyl polydimethylsiloxyethyl hexyl dimethicone (labeling name) (INCI: Triethoxysilylethyl Polydimethylsiloxyethyl Hexyl Dimethicone) produced by Shin-Etsu Chemical Co., Ltd. (Note 4) Diphenyl dimethicone/vinyl diphenyl dimethicone/silsesquioxane crosspolymer (labeling name) (INCI: Diphenyl Dimethicone/Vinyl Diphenyl Dimethicone/Silsesquioxane Crosspolymer) produced by Shin-Etsu Chemical Co., Ltd. (Note 5) PEG-9 polydimethylsiloxyethyl dimethicone (labeling name) (INCI: PEG-9 Polydimethylsiloxyethyl Dimethicone) produced by Shin-Etsu Chemical Co., Ltd. | | |

The obtained mascara was excellent in the use feeling, finish, adherence, and sebum resistance, and exhibited the effect of improving mascara smudging.

### [Example 19]

### Gommage

### <Preparation of Cosmetic>

A: Components 5 to 6 were uniformly mixed.
B: Components 1 to 4 were uniformly mixed.
C: The mixture of B was added to the mixture of A, and they were uniformly mixed to obtain a gel.

| | Component | Mass% |
|---|---|---|
| 1. | Aqueous dispersion of Example 3 (60%) | 15 |
| 2. | Phenoxy ethanol | 0.3 |
| 3. | Glycerin | 8 |
| 4. | Acrylates/C10-30 alkyl acrylate crosspolymer (3% aqueous solution) (3%) | 60 |
| 5. | Stearyl trimethylammonium chloride | 5 |
| 6. | Water | Remaining amount |
| | Total | 100 |

The obtained gommage was excellent in the finish, use feeling, and adherence, and exhibited an excellent gommage effect such as removal of sebum.

### [Example 20]

### Facial Mask

### <Preparation of Cosmetic>

A: Components 1 to 5 were uniformly mixed.
B: Components 6 to 8 were uniformly mixed.
C: Components 9 to 12 were uniformly mixed.
D: The mixtures of B and C and remaining components were added to the mixture of A, and they were uniformly mixed to obtain a facial mask.

| | Component | Mass% |
|---|---|---|
| 1. | Bentonite | 6 |
| 2. | Sorbitol liquid (70%) | 31 |
| 3. | Pentylene glycol | 3 |
| 4. | Kaolin | 0.1 |
| 5. | Titanium oxide | 2 |
| 6. | Purified water | 5.0 |
| 7. | Aqueous dispersion of Example 3 (60%) | 25.0 |
| 8. | Polysorbate 60 | 0.2 |
| 9. | Purified water | 25 |
| 10. | Sodium hyaluronate | 0.1 |
| 11. | Arbutin | 0.1 |
| 12. | Dipotassium glycyrrhizate | 0.1 |
| 13. | Citric acid (10%) | 1 |
| 14. | Sodium citrate (10%) | 1 |
| 15. | Phenoxy ethanol | 0.3 |
| | Total | 100 |

The obtained facial mask was excellent in the finish, use feeling, and adherence, and exhibited an excellent facial pack effect such as removal of sebum.

## Claims

1. An aqueous dispersion of silicone rubber particles, comprising
(A) the silicone rubber particle being an addition reaction product of (A-1) an organohydrogenpolysiloxane and (A-2) an organopolysiloxane having a monovalent olefin hydrocarbon group, wherein at least one of components (A-1) and (A-2) has a monovalent aliphatic saturated hydrocarbon group having 6 to 30 carbon atoms, bonded to a silicon atom, in a percentage of 5 to 70%, based on the total number of all substituents each bonded to a silicon atom,
in an amount of 5 to 80 % by mass, based on a total mass of the aqueous dispersion;
(B) at least one surfactant in an amount of 0.01 to 15 % by mass, based on a total mass of the aqueous dispersion; and
(C) water in an amount of 19 to 94 % by mass, based on a total mass of the aqueous dispersion.

2. The aqueous dispersion of the silicone rubber particles according to claim 1,
wherein component (A-1) is an organohydrogenpolysiloxane having two or more hydrogen atoms each bonded to a silicon atom in one molecule and represented by an average composition formula (1):
R¹ₐH_{b}SiO_{(4-a-b)/2} (1)
wherein R¹ is, independently of each other, an unsubstituted or substituted monovalent hydrocarbon group having 1 to 30 carbon atoms free from an aliphatic unsaturated bond, and ***a*** and ***b*** are the number satisfying equations 0 < a < 3, 0 < b < 3, and 0.1 ≤ a + b ≤ 3;
component (A-2) is an organopolysiloxane having two or more olefin hydrocarbon groups in one molecule and represented by an average composition formula (2):
R²_{c}R³_{d}SiO_{(4-c-d)/2} (2)
wherein R² is, independently of each other, an unsubstituted or substituted monovalent hydrocarbon group having 1 to 30 carbon atoms free from an aliphatic unsaturated bond, R³ is, independently of each other, a monovalent olefin hydrocarbon group having 2 to 6 carbon atoms, and ***c*** and ***d*** are the number satisfying equations 0 < c <3, 0 < d ≤ 3, and 0.1 ≤ c + d ≤ 3,
wherein the amount of component (A-2) relative to that of component (A-1) is an amount such that a ratio of the number of olefin hydrocarbon groups in component (A-2) per number of hydrogen atom bonded to a silicon atom in component (A-1) is 0.5 to 2, and that at least one of components (A-1) and (A-2) has the monovalent aliphatic saturated hydrocarbon group having 6 to 30 carbon atoms, in a percentage of 5 to 70% of the total number of all substituents each bonded to a silicon atom,
provided that the combination of the organohydrogenpolysiloxane (A-1) having only two SiH groups and the organopolysiloxane (A-2) having only two olefin hydrocarbon groups is excluded.

3. The aqueous dispersion of the silicone rubber particles according to claim 1 or 2, wherein component (A-1) has the monovalent aliphatic saturated hydrocarbon group having 6 to 30 carbon atoms each bonded to a silicon atom, in a percentage of 5 to 70% of the total number of all substituents each bonded to a silicon atom.

4. The aqueous dispersion of the silicone rubber particles according to any one of claims 1 to 3, wherein the silicone rubber particle (A) is a cured product of a silicone composition comprising the organohydrogenpolysiloxane (A-1), the olefin hydrocarbon group-containing organopolysiloxane (A-2), and a platinum group metal-based catalyst (A-3).

5. The aqueous dispersion of the silicone rubber particles according to any one of claims 1 to 4, wherein component (A) is the silicone rubber particle having a volume average particle diameter of 0.1 to 100 µm.

6. The aqueous dispersion of the silicone rubber particles according to any one of claims 1 to 5, wherein the silicone rubber particle (A) may absorb 30 parts by mass or more of hydrocarbon oil or ester oil, relative to 100 parts by mass of the silicone rubber particles.

7. The aqueous dispersion of the silicone rubber particles according to claim 6, wherein the hydrocarbon oil is squalene.

8. The aqueous dispersion of the silicone rubber particles according to claim 6, wherein the ester oil is ethylhexyl palmitate.

9. The aqueous dispersion of the silicone rubber particles according to any one of claims 1 to 8, wherein the silicone rubber particle (A) has no organopolysilsesquioxane on a surface.

10. A cosmetic comprising the aqueous dispersion of silicone rubber particles according to any one of claims 1 to 9.
